# EUROPEAN PATENT SPECIFICATION

(11) **EP 1 519 721 B1**
(45) Date of publication and mention of the grant of the patent: **12.03.2008**
(21) Application number: 03738845.1
(22) Date of filing: 04.07.2003
(51) Int. Cl.: G01N 33/543, B01J 20/22

(54) **POLYMER AFFINITY MATRIX, A METHOD FOR THE PRODUCTION AND USE THEREOF**
POLYMERAFFINITÄTSMATRIX, VERFAHREN ZU IHRER HERSTELLUNG UND VERWENDUNG
MATRICE D'AFFINITE POLYMERE, METHODE DE PRODUCTION ET D'UTILISATION ASSOCIEES

(30) Priority: 08.07.2002 SE 0202114
(43) Date of publication of application: 06.04.2005
(73) Proprietor: Gambro Lundia AB, 220 10 Lund (SE); Rapp, Wolfgang, 72070 Tübingen (DE)
(72) Inventor: RAPP, Wolfgang, 72070 Tübingen (DE); DEPPISCH, Reinhold, 72379 Hechingen (DE); GÖHL, Hermann, 72406 Bisingen (DE); WITTNER, Bernd, 72379 Hechingen (DE); BECK, Werner, 72108 Rottenburg (DE)
(74) Representative: Henriksson, Dan Ragnar Mikael
(86) International application number: PCT/SE2003/001166
(87) International publication number: WO 2004/004707

(56) References cited:
- EP-A2- 0 295 073
- WO-A1-01/23413
- WO-A1-92/04384
- WO-A1-92/11847
- WO-A1-98/32790
- WO-A2-00/31536
- SOKO KASAI ET AL.: 'Design and synthesis of antiangiogenic/heparin-binding arginine dendrimer mimicking the surface of endostatin' BIOORGANIC & MEDICINAL CHEMISTRY LETTERS vol. 12, March 2002, pages 951 - 954, XP002967992
- BAYER ERNST ET AL.: 'Synthese von immobilisierten peptidfragmenten an polystyrol-polyoxyethylen zur affinitaetschromatographie' ZEITSCHRIFT FUER NATURFORSCHUNG vol. 42, no. 4, 1987, pages 455 - 460, XP002967993

## Description

### Technical field

The present invention relates to a polymer affinity matrix for binding one or more substances in a fluid for removing said substance(s) from the fluid and/or decreasing the amount or concentration thereof in said fluid with a view to preventing, eliminating, or reducing undesired activation of components or processes in said fluid, to a method for removing said substance(s) from the fluid and/or decreasing the amount or concentration thereof in said fluid, to a method for producing said matrix, to use of said matrix and to a kit comprising said matrix.

### Background of the invention

### Extracorporeal treatment

Extracorporeal treatment of a fluid, such as blood or any other body fluid, requires that the fluid is brought into contact with material in the form of e.g. tubes, lines, beads or membranes. Introduction of such material implies the use of foreign and thus potentially bioincompatible (e.g. immunologically active or procoagulatory) material. This use of foreign material is associated with activation of the host immune system, e.g. of components in the blood such as lymphocytes, platelets or different types of plasma protein cascades such as proteins in the complement or coagulation cascade. Also, damage to cells such as mechanical or stress-induced damage to e.g. erythrocytes might cause haemolysis and subsequent life-threatening complications to the patient. Treatment of a fluid, such as blood or any body fluid, therefore requires the use of highly biocompatible material to avoid undesired activation of components in said fluid, e.g. blood.

### Bacterial toxins

Bacterial endo- and exotoxins promote an overwhelming inflammatory immune reaction in a host due to activation caused by multiple interactions between blood cells and soluble proteins in the host and said endotoxin. This immune response is described as an essential feature in the clinical symptoms of SIRS (systemic inflammatory response syndrome), sepsis or septic shock. The pathogenesis is severe and the condition leads to tissue damage, multiple organ failure, and death induced by sepsis. Searching for new therapeutical drugs and methods for the treatment of septic patients is important, since studies of recent therapeutical interventions (Dinarello et al. in European Cytokine Network 1997; 8:294) show a failure to protect patients with severe sepsis or septic shock. Also, when manufacturing therapeutical substances or fluids, care must be taken that the product is non-pyrogenic, i.e. that the endotoxin concentration is absent or below accepted limits for excerting its effects.

### Endotoxins

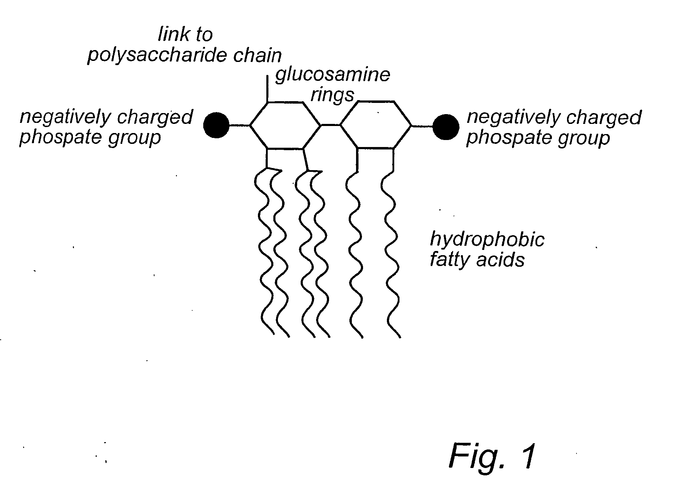

Endotoxins or "pyrogens" from the outer layers of the cell membrane of Gram-negative bacteria, e.g. Esherichia coli, Salmonella typhi, Pseudomonas aeruginosa or Proteus vulgaris, play an important role in the pathogenesis of sepsis, septic shock and systemic inflammatory response syndrome (SIRS). Endotoxins, e.g. lipopolysaccharide (LPS) from E. coli, are composed of a lipid A and a polysaccharide chain (Zähringer et al. Adv. Carb. Chem. Biochem., 1994). The molecular structure of LPS is shown in Fig. 1. The lipid A component is the biologically most active part and mediates the toxic effect of endotoxins on cells. Lipid A is highly conserved within the different strains of Gram-negative bacteria whereas the polysaccharide part is much more variable.

Lipid A from E. coli consists of two glucoseamine moieties, which contain two negatively charged phosphate groups at opposite ends of the two glucoseamines, and six long-chain highly hydrophobic fatty acid residues which are linked to the two glucoseamine rings. The variable polysaccharide chain of the endotoxin is also linked to one of the glucoseamines.

### Removal of endotoxins

The removal of endotoxins is difficult and often includes problems with recovery of or damage to valuable proteins, i.e. proteins not intended to be removed, or biocompatibility problems with the blood or body fluid and the means used for removal of the endotoxin. Such a biocompatibility problem is caused by a mere activation of the defence mechanisms of the host and involves multiple cellular activation and release of soluble proteins such as cytokines and proteins in the complement cascade. The cellular activation and protein release may lead to severe inflammation, i.e. systemic sepsis or septic shock, with tissue damage and organ failure as a result. Blood clotting caused by coagulation is another problem caused by bioincompatibility. Moreover, the pyrogen might not be completely removed.

Known methods for removing endotoxins include inactivation by the use of heat, acid or alkali (U.S. patents Nos. 3,644,175, 3,659,027, and 4,070,289). Such methods often compromise the quality of the final product, i.e. the inactivated detoxified fluid, since the fluid and its valuable proteins might be denatured or inactivated as well using this type of processes. Other methods to be used include adsorption to charcoal, or oxidative decomposition using an oxidising agent, e.g. potassium permanganate, aqueous hydrogen peroxide, and sodium hypochlorite.

### Conventional means for removal of endotoxins

Extracorporeal removal of endotoxins from plasma or whole blood of septic patients is discussed as a potential therapeutic strategy in the treatment of sepsis, septic shock and SIRS. In human plasma there are several binding proteins that play a role in the mediation and inhibition of endotoxin effects on cells, e.g. lipopolysaccharide binding protein (LBP), bactericidal permeability increasing protein (BPI), sCD14, CAP18, and lactoferrin. The peptide antibiotic polymyxin B (produced by Bacillus *polymyxa)* inhibits the action of endotoxins on cells. The horse shoe crab *(Limulus polyphemus)* also contains endotoxin-binding proteins and a cell lysate from this species is used for the detection of endotoxins (Limulus amebocyte lysate, LAL, or Limulus test). The binding motifs of such endotoxin binding proteins may be used for extracorporeal treatment of plasma or whole blood of septic patients.

A common characteristic of many endotoxin binding sequences is the presence of alternating positively charged and hydrophobic amino acids in the binding part thereof. It has been shown for one endotoxin binding protein originating from the horse shoe crab that the endotoxin binding sequence forms an amphipatic secondary structure where positively charged residues and hydrophobic residues are located at opposite faces of a loop structure (Hoess et al. EMBO J, 1993). A similar pattern has been proposed for other endotoxin binding sites.

Positively charged polymers such as polyethyleneimine (Mizner et al. Artif. Organs, 1993; Weber et al. ASAIO J, 1995; Petsch et al. J Chromatogr. Biomed. Sci. Appl., 1998) or diethylaminoethyl (DEAE) modified cellulose (Weber et al. A.S.A.I.O. J., 1995) have a certain adsorption capacity for endotoxins, probably based on the interaction of the positive charges with the negatively charged phosphate residues of lipid A. A drawback of polyethyleneimine is the high absorption of heparin and its well-described interaction with platelets which gives rise to bioincompatibility problems such as coagulation in an in or ex vivo application.

Similarly, positively charged membrane filters are used for purification of infusion fluids. Absorption is thus dependent on attractive charges and is less specific and may therefore remove desired valuable proteins as well.

Arginine immobilised on sepharose has been suggested for removal of endotoxins from plasma, blood and pharmaceutical solutions (EP 0 494 848 and EP 0 333 474).

WO 92/11847 describes the use of a compound for the preparation of a medicament to be used orally, intravenously, intramuscularly, intracutaneously or intraperitoneally for the treatment of endotoxin induced effects as well as a method for the treatment of endotoxin induced effects. The compound described in WO 92/11847 is not immobilised on a solid support.

WO 92/04384 A1 discloses use of different spacers for providing defined distances between a solid phase and a ligand in a method for inhibition of endotoxin induced effects.

WO 01/23413 discloses ligands bound to a solid support via a spacer. Said ligand consists of a mixture of linear or branched oligopeptides and is effective for adsorption of a large variety of endotoxins.

Bioorganic & Medicinal Chemistry letters 12 (2002) 951-954, Soho Kasai et al, "Design and Synthesis of Antiangiogenic/Heparin-Binding Arginine Dendrimer..." discloses TX-1943 and TX-1944, consisting of arginine and lysine residues bound to a Wang resin via a glycine.

Also, hydrophobic polymers (e.g. polystyrene, polyamide, polysulfone, and polyethersulfone) can adsorb endotoxins in aqueous solutions. This property is used in ultrafiltration membranes for purification of water and dialysis/infusion fluid, i.e. solutions with low a or no protein content (Weber et al., Int. J. Artific. Org., 1997). However, the removal of endotoxins from blood or plasma introduces a competing problem with circulating plasma proteins (LBP, BPI, sCD14) and of cellular receptors (e.g. CD14) to an adsorber matrix due to the plain hydrophobic absorption which has a low specificity.

The use of positively charged or hydrophobic polymers for the removal of endotoxins shows that the specificity of the binding and thus also the selectivity for the removal of the endotoxin in plasma is low. In practice it has been a challenge to develop an efficient endotoxin adsorbent with both high specificity and high selectivity, still in combination with high biocompatibility.

The use of peptide sequences from endotoxin binding proteins has been suggested for therapeutical applications (US patents Nos. 5,639,727 and 5,643,875) by use of *e.g.* bactericidal/permeability-increasing (BPI) protein products. A drawback of immobilised peptides is the costs of synthesis and the necessity of the peptides to be immobilised without interfering with the binding structure.

Affinity ligands such as histamine, histidine and polymyxin B are effective for the removal of endotoxins though their effectiveness is dependent upon other proteins in the fluid and decreases drastically in the presence of serum proteins like serum albumin or other negatively charged proteins (Anspach and Hilbeck, J. Chromatogr., 1995, Petsch et al. J. Chromatogr., 1997, EP 0 129 786). However, Polymyxin B is toxic to the central nervous system and may cause kidney damage, which is a drawback in marketing approval due to a risk of leakage into the blood of a patient. US 4,771,104 describes an endotoxin detoxifying material comprising a fibrous carrier to which polymyxin B is fixed.

Removal of endotoxins, particularly LPS, from drugs and fluids by use of water-insoluble poly(ε-lysine) (PL) particles was described by Hirayama et al, in Journal of Chromatography B, 271 (1999), 187-195.

### Limitations and future perspectives

Efficient endotoxin removal from a protein fluid is dependent on the net charge of the desired valuable protein from which the endotoxin should be removed. The interaction between the endotoxin and its ligand is of both hydrophobic and ionic character, though contribution of each of them depends on the ionic strength and pH of the fluid. Efficient endotoxin removal is also dependent on a high perfusion and biocompatibility of the means for removal of said endotoxin to prevent e.g. cell activation or blood clotting.

However, although many means have been developed or suggested for removal of substances such as endotoxins from fluids, e.g. blood, other body fluids or therapeutical fluids, none of the described means is highly biocompatible nor has a high degree of specificity and selectivity against substances to be removed e.g. endotoxins, and at the same time can be easily perfused, e.g. by whole blood or plasma. It is thus desirable to develop highly efficient, biocompatible, and effective methodologies and means for the removal of substances, e.g. endotoxins, from a fluid, and thus making it possible to avoid the problems associated with prior art devices. In this respect, the present invention addresses this need and interest.

The need for a novel and efficient method and means for decreasing the concentrations of or removing one or more substances from a fluid, e.g. endotoxins from blood, any other body fluid or therapeutic fluid with a view to preventing, eliminating or reducing undesired activation of components or processes in a fluid is evident from the reasons described above, particularly within the biomedical area. Such a method and means would also be specifically valuable in the treatment of sepsis, septic shock and SIRS by extracorporeal removal of pyrogens or other activating substances from plasma of septic patients.

Further, it is well known to bind a biospecific ligand e.g. an antibody or a peptide to a matrix or substrate and remove by this e.g. pathophysiologically critical substances from the body or blood circulation.

Used known materials are e.g. sepharose (sephadex, pharmacia), polyacrylate or epoxid resins in form of beads or particles.

Known beads are for example made of polymethylmethacrylate e.g. DALI-System (Fresenius) for LDL adsorption; Sepharose: e.g. Rheosorb-System by Plasmaselect for fibrinogen adsorption which uses an immoblized peptide; Therasorb-Immunoadsorption-System to remove autoantibodies using an immobilized sheep-antibody which binds human immunoglobulins; Excorim-Protein A column (and similar systems by other manufacturers) which uses an immobilized bacterial protein to bind immunoglobulins.

Known membranes are for example polyamide (MAT/Merck) and others usually described as affinity membranes.

Known fabrics are for example Toray polystyrene/polyethylene meshes with Polymyxin B ligand for LPS binding.

All these substrates are modified in case of use of biological ligands (such as peptides or antibodies.) by conventional methods of wet chemistry, i.e. first the corresponding specific ligand is formed or isolated (e.g. by peptide synthesis, by biotechnological methods), then it is purified and afterwards it is immobilized to a solid matrix.

These matrices usually do not allow solid phase synthesis of a ligand (e.g. a peptide) directly on the matrix, due to chemical incompatibility against the chemicals (solvents, acids and bases used for cleavage of protecting groups etc) used in solid phase synthesis.

The matrices that are suitable for solid phase synthesis (e.g. polystyrene) are not suitable for therapeutic purposes due to lack of biocompatibility.

Therefore, a solid phase matrix, which allows solid phase synthesis as well as contact with blood components (e.g. plasma or whole blood), would be advantageous for the following reasons:
(1) The development steps to find a suitable ligand (e.g. an optimised peptide structure) can be performed on the same solid matrix which can later be used for therapeutic application. This means that the necessary biological test procedures (e.g. measurement of affinity, specificity and binding capacity) can be performed under conditions very similar to the conditions of the later therapeutic application. By this the information coming from the biological test procedures is very reliable and predictive for the later therapeutic or clinical application. This saves time and money and lowers risk of failure or side effects.
(2) A (peptide) ligand can be built-up by solid phase synthesis in an exactly defined way (with respect to sequence and geometry). This means also that the linkage (i.e. the location of the covalent connection) of the (peptide) ligand to the solid matrix is exactly defined.

There are somehow contradicting requirements to such a matrix with respect to the swelling or wetting behaviour: for the solid phase synthesis usually nonaqueous organic (polar, such as dimethylformamide, or apolar, such as dichloromethane) solvents or mixtures of solvents are used. On the other hand the therapeutic application must take place in an aqueous environment (e.g. plasma, blood, therapeutic solutions etc). In both environments (i.e. aqueous and organic solvents) the polymer matrix must be easily wettable and swellable:
(1) in order to be able to effectively remove or rinse out unbound (excess) ligand or building blocks (e.g. protected amino acids) or chemicals (e.g. coupling agents, such as carbodiimides, deprotection agents, such as organic acids or bases), and
(2) in order to allow the respective toxin to be removed from the aqueous environment, the matrix must be wettable and swellable in this environment, too.

Accordingly, the problem with known polymer matrices are that you are restricted to the type of regenerating fluid to use in order not to elute the bound active ligand as such but only the in the material caught substances to be removed from the fluid, and the ligand is build up as a defined peptide/molecule or a randomly polymerised polypeptid/oligomer and has then to be bound to the support material as such - you do not know what you end up with in the end.

The advantages with the use according to the invention are that you can build the ligand directly on the solid support with the grafted polyethylene glycol and the solid support with the built ligand could be used directly. This provides for reliability which you do not get with prior art matrices. Further, it provides for technological advantages as the biologically active/specific ligand is part of the medical device and the development time is shortened (this could allow or facilitate a kind of individualized treatment).

During the development the material according to the invention was not known or expected to work so brilliantly as it turned out to do. The expected drop backs was e.g. that polymer matrix would swell and plug the flow through the device. Further, the pressure drop when applying a fluid like whole blood was expected to be too high. Finally, as a result of the matters above, the perfusion of the fluid through the solid phase material was expected to be insufficient.

The polymer matrix according to one of the preferred embodiments has shown to be excellent for steam sterilisation and to thereafter be dried. After drying the residue of air is easily removed by adding a saline solution for the polymer matrix to swell again and being ready for use. This leads to short preparation time in clinic, which is important e.g. in emergencies and in times of high workload.

Further, the material shows instant wetting, which is not the case with most used solid supports used for different types of ligands. This is a very important feature as a solid phase material for active ligands. Even further the material is able to be compressed and decompressed within a certain range, and the material is biocompatible with respect to complement activation, contact phase activation, cytotoxicity and granulocyte activation.

### Summary of the invention

Therefore, the main object of the present invention is to eliminate the problems associated with the prior art by providing a highly biocompatible, specific, selective and easily perfused polymer affinity matrix for removing and/or decreasing the amounts or concentrations of the above-mentioned undesired substances, e.g. endotoxins, in fluids, i.e. a polymer affinity matrix having all the advantages of the prior art and none of the disadvantages.

This object is achieved according to a first aspect of the present invention, i.e. with a polymer affinity matrix for binding one or more substances in a fluid for removing said substance(s) from the fluid and/or decreasing the amount or concentration thereof in said fluid with a view to preventing, eliminating, or reducing undesired activation of components or processes in said fluid, wherein said matrix comprises the components a) - c) defined in claim 1.

In a preferred embodiment the present invention relates to a polymer affinity matrix for removal of endotoxins to decrease the undesired activation of components or processes in a fluid, wherein the matrix provides a ligand having a three-dimensional structure complementary to the three-dimensional structure of a binding motif of said endotoxin, thereby allowing binding of the endotoxin.

In the preferred embodiment for the removal of endotoxins to decrease the activation of the fluid, each binding unit in the polymer affinity matrix is arginine. Such a polymer affinity matrix generates a defined cut-off of about 1x10²-1x10⁶ Daltons and can be used for binding both hydrophobic and/or hydrophilic substances.

In another aspect the present invention relates to a method for removing one or more substances from a fluid and/or reducing the amount or concentration thereof in said fluid with a view to preventing, eliminating or reducing undesired activation of components or processes in said fluid, comprising contacting the fluid with the polymer affinity matrix according to the invention for a period of time sufficient to reduce the amount or concentration and/or remove said substance(s) of interest, preferably up to 24 hours, most preferably from 1 s to 2 hours. However, the period depends on the flow rate, column size and mode of application, i.e. if the treatment is made in vivo, ex vivo or in vitro. Preferably, the amount or concentration of said substance(s) after having been removed or reduced is below the capacity of activating components or processes in blood or prevents activation of components or processes in blood.

In a further aspect the invention relates to a method for producing the polymer affinity matrix as defined in any one of claims 1-10 according to the present invention, comprising the following steps:
a) attaching the spacer to the solid support to obtain a first complex and
b) attaching to said first complex the ligand containing said at least one binding unit with at least one functional group,
   or
c) attaching the spacer to the ligand containing said at least one binding unit with at least one functional group to obtain a second complex, and
d) attaching the solid support to said second complex,
   or
e) attaching the spacer to the solid support to obtain a first complex, and
f) solid phase synthesis of the ligand on the spacer bound to the solid support,
   or
g) building up or synthesizing the spacer from monomers directly on the solid support by grafting, and
h) attaching to said first complex the ligand containing said at least one binding unit with at least one functional group,
   or
i)building up or synthesizing the spacer from monomers directly on the solid support by grafting, and
k) solid phase synthesis of the ligand on the spacer bound to the solid support,
wherein information about the three-dimensional structure, presence of charges and hydrophobic/hydrophilic regions of the binding motif on the substance(s) to bind is collected from X-ray crystallography, protein sequencing, protein modelling or hydrophobicity and hydrophilicity calculations and the binding unit is made complementary as regards charge and/or hydrophilicity/hydrophobicity to the binding motif of said substance(s).

In still a further aspect the present invention relates to use of the polymer affinity matrix for removal of one or more substances, preferably endotoxins, from a fluid, or decreasing the amount or concentration thereof in said fluid, preferably a body fluid or a therapeutic fluid, most preferably blood.

In still a further aspect, the invention relates to a kit for removing one or more substances from a fluid and/or decreasing the amount or concentration thereof in said fluid with a view to preventing, eliminating, or reducing undesired activation of components or processes in said fluid, said kit comprising the polymer affinity matrix, sample tubes, and a device for extra- and/or intracorporeal treatment of said fluid, preferably blood or serum.

The use of a polymer affinity matrix according to the present invention will optimise the treatment of fluids, such as blood, any other body fluid or therapeutic fluids for removal of one or more substances, such as endotoxins, and/or reducing the amount or concentration thereof with a view to preventing, eliminating or reducing undesired activation of components or processes in said fluid.

Specifically, the use of a highly biocompatible and perfusable material according to the invention is important for prevention of the activation in the fluid during use of the polymer affinity matrix. This is of particular importance at extracorporeal removal of endotoxins from plasma or blood of septic patients and is disclosed as a potential therapeutic strategy in the treatment of sepsis, septic shock and SIRS. As a further advantage, the use of the polymer affinity matrix according to the present invention will reduce the treatment time for the patient.

Further advantages and features of the present invention will become more apparent from the following detailed description when taken in conjunction with the drawings and the appended claims.

### Brief description of the drawings

Fig. 1 is a schematic view of a lipid A moiety of the LPS molecule with its structural elements.
Figs. 2A and 2B are schematic views of examples of ligands belonging to the tree-like structure (according to the present invention) (2A) and the comb-like structure (2B) of the polymer affinity matrix.
Fig 2C shows examples of polymer affinity matrixes having a cyclic ligand structure.
Figs 3A-3B show how the complementary structure of the endotoxin is generated by using structural requirements and positive charges, hydrophobic regions and optimal distances in the binding motif.
Fig. 4 describes different ways of producing a polymer affinity matrix according to the present invention.
Fig. 5 shows levels of endotoxin in human plasma after incubation with PS-PEG-beads for different time points (1, 10 and 120 minutes).
Figs 6A-6G show the inhibition of endotoxin induced production of intracellular IL6 in monocytes after treatment of LPS spiked blood with PS-PEG-Arg₈ beads in comparison to beads without the matrix, i.e. reference material (PS-PEG-reference material).
Figs 7A-7D show the biocompatibility profile of PS-PEG-Arg₈ beads measuring white blood cell (WBC) and red blood cell (RBC) numbers, thrombocyte (THR) numbers and hematocrit (HCT).
Fig. 8 shows the lack of TCC (terminal complement complex) formation after use of PS-PEG-Arg₈ beads in whole blood.
Fig. 9 shows the absence of elastase release after use of PS-PEG-Arg₈ beads in whole blood.
Fig. 10. shows the absence of TAT (thrombin-antithrombin III complex) formation after use of PS-PEG-Arg₈ beads in whole blood.
Figs 11A-11B show a three-dimensional structure of the ligand containing the binding unit of -Arg₈ and -Arg₄ optimised for LPS binding.
Fig. 12A shows curve-fitted Langmuir isotherms for PS-PEG-Arg 8 and PS-PEG-Arg 4 related to mass of beads in gram(s).
Fig. 12B shows curve-fitted Langmuir isotherms for PS-PEG-Arg 8 and PS-PEG-Arg 4 related to moles of arginine.

### Detailed description of the invention

As indicated above, the present invention relates to a polymer affinity matrix for removal of substances from a fluid, such as blood, any other body fluid or therapeutic fluids, to decrease the activation of components or processes in the fluid, while at the same time having sufficient specificity to avoid adsorption of other valuable substances in the fluid, e.g. physiological components of blood. Said matrix provides a ligand having a structure that is complementary to the structure of a binding motif of the substance to remove or reduce the amount or concentration of, e.g. an endotoxin.

### Definitions

In the present context, the term "removing ... a substance" is intended to mean preventing, reducing, decreasing, neutralising, inactivating, degrading, modifying, scavenging, binding or concealing a substance, not always intended to mean to a zero amount or concentration of the substance. Moreover, the term "decreasing the amount or concentration of a substance" is intended to mean a reduction or decrease of the amount or concentration of a substance in a fluid enough to prevent or inhibit subsequent activation of components in the fluid and/or cellular and/or non-cellular biological mechanisms in the fluid.

With the term "preventing, eliminating, or reducing undesired activation of components or processes" the meaning is to inactivate, inhibit, lower, decrease, reduce, slow down or prevent a certain chemical, biological or biochemical process in the fluid e.g. in blood or tissue cells, e.g. plasma protein cascades such as signal transduction pathways, complement or coagulation processes and/or the activation of components involved in such processes, in a direct or indirect way. A "direct" removal of a substance influences the procedure without any intermediate steps, whereas an "indirect" removal implies removal of a substance being part of a long chain or cascade of reactions, where removal of a substance early will prevent, slow down or inhibit a downstream event of interest. This means that the removal of a substance from e.g. blood may give an inactivated or less activated blood. Also, implied herein is the prevention of activation of the fluid, such as blood.

The term "fluid" is intended to include any fluid, such as any gas or liquid, such as a suspension or a solution, including conventional solutions, e.g. aqueous or organic solutions, or blood, any body fluid or therapeutical fluid, fluids for life science applications such as fluids in biological, diagnostic or biotechnological applications e.g. buffer solutions, infusion fluids, or dialysis fluids, fluids for nutrition and fluids for industrial use.

The term "body fluid" is intended to include blood, plasma, cerebrospinal fluid, ascites and reinfusion fluids (e.g. after hemoconcentration), blood products obtained from healthy donors, such as plasma, platelet concentrates, erythrocyte concentrates, which are used for transfusions.

The term "therapeutical fluid" is intended to include peritoneal dialysis fluids, hemodialysis concentrates and dialysis water, substitution fluids/on line prepared fluids, infusion fluids, parenteral nutrition fluids, lavage fluids in surgical environment and fluids for blood component preparation, blood substitutes (e.g. oxygen carriers, modified hemoglobin solutions, artificial hemoglobin solutions).

The term "fluids for life science application" is intended to include fluids and/or media for cell culturing tissue engineering, molecular biology (e.g. solutions of proteins and enzymes used for PCR techniques), bacteriology, analytics and pharmaceutical preparation.

The term "fluids for nutrition" is intended to include drinking water, fluids for outdoor situations and reconstitution fluids for food and drinking concentrates or powders.

The term "solid support" is intended to mean a solid phase or support or an insoluble matrix whereupon a molecule, e.g. a ligand in the form of a polypeptide, can be synthesised or coupled with or without a linker or spacer in-between.

The term "functional group" is intended to mean a specific atom, or group of atoms, that gives a molecule, i.e. the binding unit in the matrix according to the present invention, e.g. an amino acid, a fatty acid, a carbohydrate, a lectin, and a nucleotide, and derivatives thereof, or combinations thereof, a specific chemical characteristic or structure, e.g. a positive or a negative charge, hydrophobicity or hydrophilicity, and/or any other physio-chemical force, e.g. van der Waals forces and π-π-interactions among aromatic groups or a capacity to form further bonds, such as hydrogen bonds or covalent bonds. Examples of functional groups on amino acids are -COOH, -OH, -SH, guanidino, and -NH₂, but may also be a substituted amino group or any positively charged group or mixtures thereof.

The term "binding unit" is intended to mean the molecule mentioned above under the definition of the term "functional group", wherein said molecule is responsible for the binding to the substance(s) to be removed and/or reduced, contains at least one functional group and is included in the ligand bound to a spacer in the polymer Page 19a affinity matrix according to the present invention.

Each binding unit may comprise an amino acid being positively charged at or around the physiological pH of blood, such as arginine, lysine, cysteine, or histidine, or another bi- or trifunctional molecule having at least one functional group being positively charged at or around the physiological pH of blood.

The term "binding motif" is intended to mean a three-dimensional structural and chemical motif, single or repeated, on the substance(s) to be removed.

The term "ligand" means the whole three-dimensional molecule bound to the matrix via the spacer, i.e. comprising at least one binding unit with at least one functional group. The ligand forms a three-dimensional complementary structure with and binds to the binding motif on the substance to be removed. Here, "complementary" is intended to mean a three-dimensional and geometrically defined structure characterised by a capacity for precise pairing with the three-dimensional complementary structure of a binding motif of the substance to be removed.

The term "derivatives thereof" used in connection with a certain compound means one or more compounds derivatized in such a way that they have the same or essential same function as the compound as such.

Further, isomers of the compounds constituting the ligand are also intended to be included within the scope of the invention, provided they show the same or essentially same function as the compounds as such. In the structure formulas, a and e defines, according the nomenclature commonly used for amino acids, the amino groups used for covalent coupling of further molecules.

The term "substance(s)" is intended to mean at least one component or molecule of interest, soluble or non-soluble, which is intended to be bound to the binding unit. Examples of substances are toxic substances that may activate blood cells such as substances derived from viruses and bacteria, e.g. an endotoxin, a blood cell population, such as a lymphocyte, thrombocyte, granulocyte, dendritic cell, monocyte, endothelial cell, stem cell, tumour cell; or a blood component or a product from a metabolic activity such as glucose derived molecules or degradation products thereof, blood clotting proteins, procoagulatory proteins, inflammatory or proinflammatory proteins, cytokines, growth factors, hormones, chemokines, uremic toxins, and macrophage migration inhibitory factor. Examples of blood components are infectious substances causing *e.g.* a contagious disease including virus particles, prions, or parasites, fungi, pathogen-loaded blood cells, as well as drugs after overdosing, pathogenic food additives or other components not originating from the body. Also intended to be included are pyrogens, particularly bacterial pyrogens, bacterial exotoxins, products from Gram-positive bacteria, such as lipoteichonic acid, products from metabolic disturbances, chronic or acute, as a result of e.g. diabetes mellitus, liver disease, uraemia or kidney diseases or inflammation, as well as adhesion cascades, e.g. soluble adhesion molecules. Preferred substances to be removed are e.g. endotoxins from Gram-negative bacteria, such as LPS, as well as bacterial DNA or fragments or degradation products thereof, and oligonucleotides, heparin, phosphate, blood cells, soluble or cell surface bound proteins.

The term "spacer" is intended to mean a chain-formed molecule, e.g. a polymer, which modifies the solid support in the sense of becoming a part of the solid support and positions the ligand with the at least one binding unit containing at least one functional group away from the solid support and makes it less restricted by steric hindrance from the solid support and more available to the substance(s) to bind, e.g. an endotoxin, cell population or blood component. The spacer molecule may be in a linear and/or branched and/or cyclic format. The spacer length is herein predefined from structural requirements of the substance(s) to be removed.

The term "distance molecule" is intended to mean bifunctional molecules within the ligand having the function of creating a structural distance, if desired, between the binding unit and the trifunctional branching molecule defined under general formulas I and II below and/or between the spacer and said trifunctional branching molecule. The distance molecule may also be in a cyclic format.

The term "pyrogen" and particularly "bacterial pyrogen" defines a fever-producing substance, more commonly of bacterial origin, e.g. an endotoxin.

Here, the term "biocompatibility" is intended to mean the lack of or absence of activating capacity, of e.g. activation of cells, coagulation, complement cascades or similar processes, in the sense that the use of the matrix does not lead to an activation of the immune system of the patient, or at least if such an activation occurs, it is only to a minor degree. This means that the use of a biocompatible compound or substance will not lead to any unwanted or undesired activation of components or processes in e.g. blood or other body fluids as well as mechanical or stress-induced cell death or cell lysis.

### The polymer affinity matrix

To create diversity in a polymer affinity matrix enough to bind and adsorb a large variety of substances, the ligand may comprise 1-100 functional groups, preferably 1-32 functional groups, but the ligand in the polymer affinity matrix according to the present invention has the structure defined in claim 1.

The polymer affinity matrix according to the present invention may be in the form of a bead, a gel-like structure, a membrane or part of a membrane, a film, or a net, or a combination thereof.

Specifically, when the matrix is composed according to the present invention, it will generate a defined cut-off from about 1x10² to 1x10⁶ Daltons and may bind both hydrophobic and hydrophilic substances without any restriction.

In a preferred embodiment, the polymer affinity matrix is in the form of a PS-PEG bead (e.g. TentaGel^{®}, obtainable from Rapp Polymere Tübingen), using PEG as a spacer. PEG used as a spacer has the advantage of good swelling of the beads in both organic and aqueous solvents and allows, due to its quasi-fluid properties, similar diffusion processes as in a fluid, preferably a diffusion coefficient close to water, i.e. 40%.

### The biocompatibility

According to the invention, the polymer affinity matrix should in a preferred form show a high biocompatibility when used in a specific application, such as extracorporeal treatment of whole blood. A high biocompatibility implies that certain characteristics are intended to be more important than others. For example, no complement activation, measured by early complex formation and quantitation of terminal complement complex (TCC protein) according to Deppisch et al. (Kidney Int. 37:696-706) is important. Also, a low thrombogenicity is desirable for the patient. The degree of thrombogenicity is measured by quantitation of thrombin-antithrombin III complex (TAT) according to Deppisch et al. (Neuphrol. Dial. Transplant Suppl. 3:17-23, 1994) and should not increase during blood or plasma treatment. Still, a steady blood cell number of white and red blood cells before and after treatment is included as well as an absence of cell activation due to contact phase activation. A steady blood cell count intends to mean a low degree of or virtually no damage to blood cells due to mere stress, activation or mechanical damage.

Proteases, such as elastase produced by e.g. granulocytes or neutrophiles upon activation, are increasing in patients with bacterial sepsis and septic shock (Heiden et al. Sem. Thromb. Hemost. 1996). Neutrophilic elastase is also suggested as an early and effective marker of infection (Jensen et al., Scand. J Clin. Lab. Invest, 1996; Groenenveld et al, Cytokine, 1995). Measurements of elastase may give additional information about biocompatibility, and levels should accordingly not change during blood treatment.

### The ligand

To be able to create a ligand complementary to the binding motif of the substance to be removed, such as an endotoxin, a three-dimensional structure has to be formed. This may be achieved by the use of e.g. a flexible or rigid polypeptide structure, which is easy to synthesise in an optimal way for the required three-dimensional structure. Such a polymer may be linear or branched, e.g. in a tree- or comb-like structure, or cyclic. Beneficial to the formation of such three-dimensional structures is the use of amino acids since they provide flexibility by nature. The chemistry for coupling such a polymer, e.g. a polypeptide, is well known within the art. Such a polymer refers to a polymer containing more than one amino acid, generally up to about one hundred, *i.e.* an oligomer, linked together by peptide bonds. Examples of linear polymers are poly- or oligopeptides formed by amide bonds between the alpha-carboxyl and alpha-amino groups of adjacent residues, and examples of branched polymers are poly- or oligopeptides formed by amide bonds involving one or more non-alpha-amino groups.

As stated above, the ligand molecule may be in a cyclic format. The cyclic structure may be formed by a covalent coupling between two appropriate functional groups within the ligand structure, e.g. a disulfide bond (-S-S-) between two SH groups of cysteine by oxidation (a common cyclisation reaction also occurring in natural protein structures).

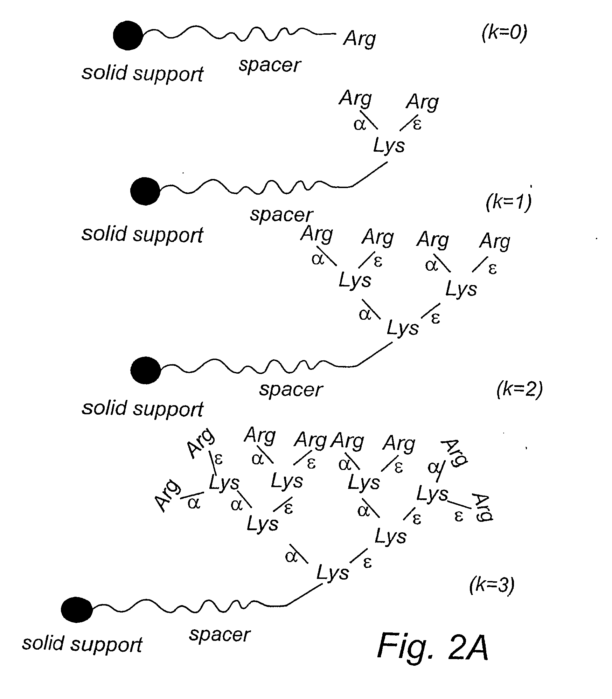

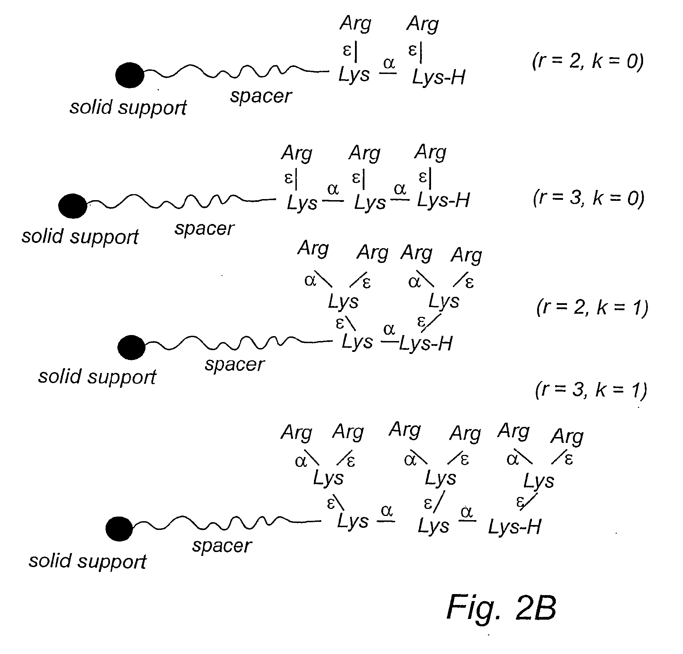

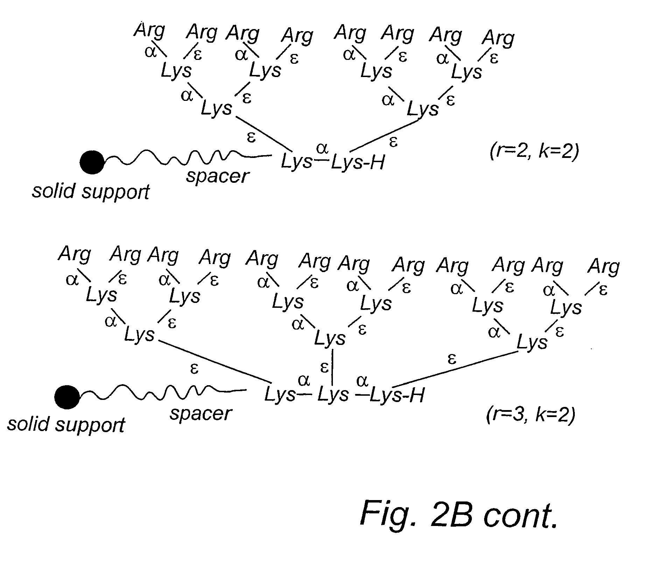

Figs. 2A and 2B show examples of different branched, tree-like and, comb-like structures, respectively, of the ligand couple to a spacer boun to a solid support, but the present invention only refers to the tree-like structures in Fig 2A. In these structures the ligands are basically built up of lysine residues, and the binding units in each ligand are arginine residues. The ligand included in the polymer affinity matrix may be represented by the two following formulas:
General Formula I:

   -X¹ₙ-Yₘ[X²ᵢ-Z¹; X³ⱼ-Z²]_{½(m+1)}
In general Formula I, representing a tree-like structure, the different symbols have the following meaning:
   n = 0 or 1;
   m = 2^{k}-1;
   k = 0 to 10, wherein if k = 0 then X₂ = X₃ and Z₁ = Z₂;
   i = 0 or 1; and
   j = 0 or 1,

General Formula II:

-(X¹ₙ-Y¹[Y²ₘ [X²ᵢ-Z¹; X³ⱼ-Z²] _{½(m+1)})ᵣ-X⁴ₚ-Z³

In general Formula II, representing a comb-like structure, the different symbols have the following meaning:
n = 0 or 1;
m = 2^{k}-1;
k = 0-10, wherein if k = 0 then X₂ = X₃ and Z₁ = Z₂;
r = 1-100;
i = 0 or 1;
j = 0 or 1; and
p = 0 or 1.
Z¹, Z² and Z³ each represents the binding unit and is each an organic molecule chosen from the group consisting of an amino acid, a peptide, a fatty acid, a carbohydrate, a lectin, and a nucleotide, and derivatives thereof, or combinations thereof, wherein Y, Y¹ and Y² each is a trifunctional branching molecule which contains functional groups chosen from the group consisting of amino, hydroxy, aldehyde, isocyanate, isotiocyanate, thiol, maleimido, epoxy, and derivatives thereof, or combinations thereof, and wherein
X¹, X², and X³ each is an optional bifunctional distance molecule containing two functional groups chosen from the group consisting of amino, carboxy, hydroxy, aldehyde, isocyanate, isothiocyanate, thiol, maleimido, epoxy, and derivatives thereof, or combinations thereof.

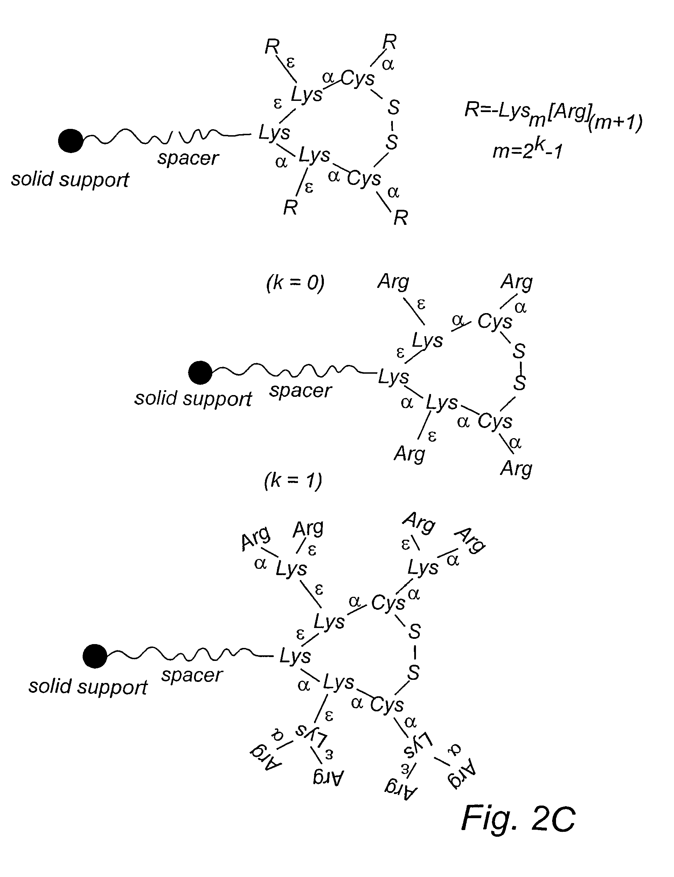

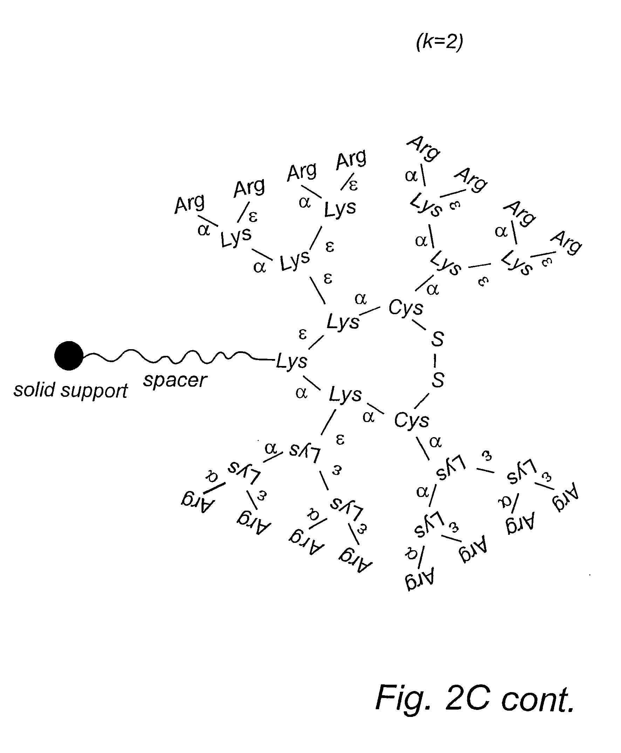

In Fig 2C examples of polymer affinity matrixes having a cyclic ligand structure are shown. In the structure formulas R means -Lysₘ[Arg]₍ₘ₊₁₎, wherein m=2^{k}-1, and examples wherein k=0,1, and 2 are shown.

Thus, the at least one binding unit of the ligand in the polymer affinity matrix contains at least one functional group, preferably an amino group or an guanidine group or a substituted amino group or at least one of said functional groups.

Preferably, the amino acids are chosen for their characteristic of being positively charged at or around physiological pH, specifically of blood ≥7.2, i.e. at a pK about ≥ 6.0. Examples of such preferred amino acids are arginine (Arg), lysine (Lys), and histidine (His). Also mixtures of said amino acids may be used in the ligand of the matrix, to create still a further variability.

Most preferably, the ligand comprises arginine as binding unit(s) and constitutes ≤ 3mmol/g matrix. In general, the amount of amino acids may be at least about 0.01-5 mmol/g matrix.

In the embodiment when the binding unit of a branched ligand comprises the amino acid arginine, the number of arginine molecules per ligand is 2-100 arginine molecules, and preferably 4-8 molecules, known as Arg₄₋₈. Still, by using a trifunctional amino acid, i.e. containing three-functional groups, here two amino groups and one carboxy group, such as lysine, a branched structure can be created. This approach of using so called multiple antigenic peptides, MAP, has been introduced by Tam et al., and is described in U.S. patent No. 5,229,490. By variation of the number of branches, the clustering and the distances of the end groups, e.g. positively charged arginine comprising terminal positive functional groups, can be varied, as discussed above in connection with Fig. 2.

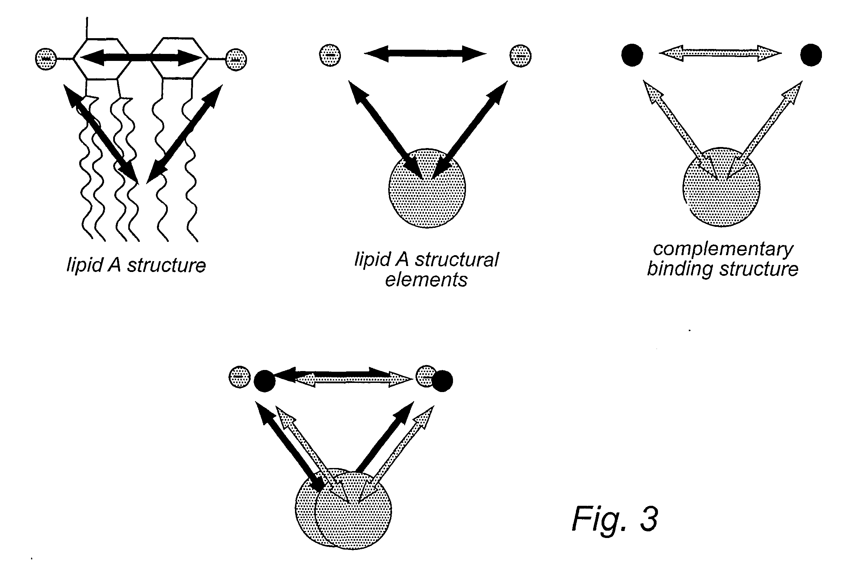

In a specific embodiment of the invention, where endotoxins are the substances to be removed by the polymer affinity matrix, the positive charges of the functional groups of the amino acids are held at a distance defined by the distance between individual negatively charged phosphate groups in an endotoxin as shown in Fig. 3.

As discussed above, the amount and the configuration of the amino acids create the variability of the complementary ligand in the polymer affinity matrix, and may therefore vary to create sufficient variability for absorbing a large variety of substances. In another embodiment of the invention the amount of amino acid is at least about 0.01, 0.1, 1, 2, 3, 4, or 5 mmol/g matrix.

### Design of the ligand containing the binding unit(s)

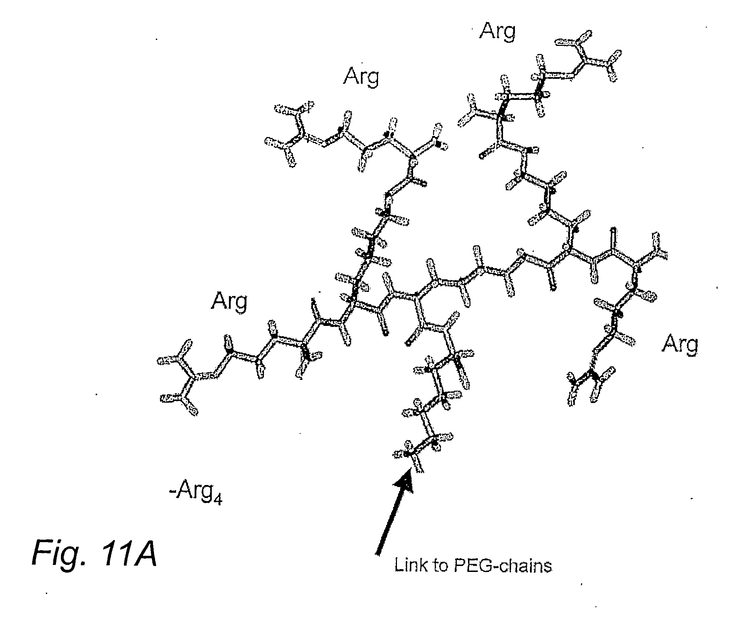

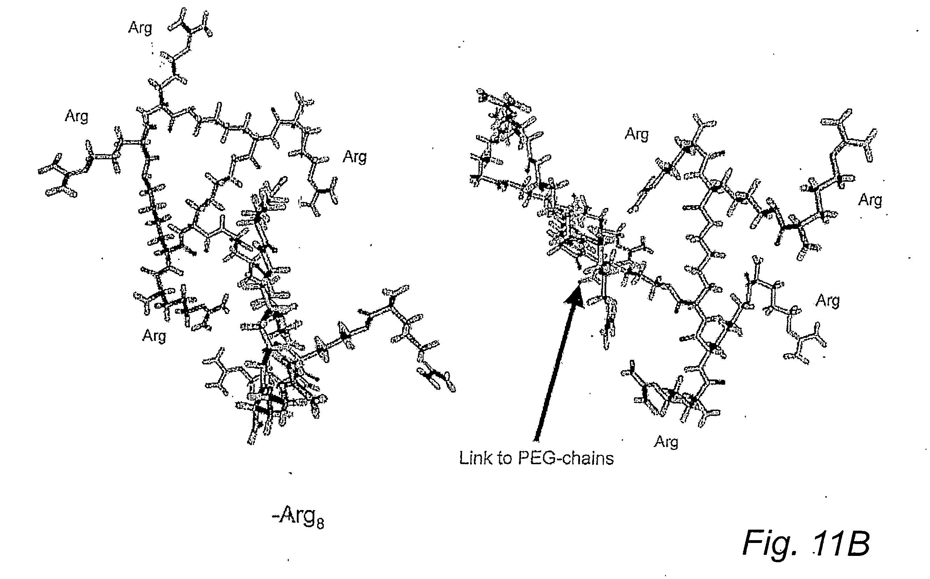

To achieve a complementary structure to a substance, information is collected from procedures known in the art for studying the relationships regarding structure, presence of positive charges being held at a certain distance as well as presence of a hydrophobic region in proximity to the charged groups, e.g. X-ray crystallography, protein sequencing, protein modelling or hydrophobicity and hydrophilicity calculations as described in Example 1 and shown in Figs 3 and 11.

In the preferred embodiment, the substance to be removed is an endotoxin, such as LPS containing lipid A, and information known to the skilled man in the art about the molecule regarding e.g. structure and distances between charged phosphate groups within the molecule is used to form a ligand with at least one binding unit in the polymer affinity matrix. The polymer forming the ligand is in this embodiment synthesised by amino acids, e.g. arginine and/or lysine.

In the Arg₄₋₈ embodiment of the invention as the complementary part to the binding motif of the substance to remove, e.g. an endotoxin, said arginine as binding units possess the following properties for the removal of the endotoxins as shown in Fig. 3:
a) presence of positive charges being held at a distance which optimally fits with the distance of the negatively charged phosphate group in lipid A,
b) presence of a hydrophobic region in proximity to the charged groups allowing hydrophobic interactions with the fatty acid moieties of lipid A, and
c) a combination of said properties in a) and b) to give a complementary structure to lipid A allowing optimal binding.

### The spacer

The polymer affinity matrix according to the invention comprises a spacer that is substantially hydrophobic or hydrophilic, modifying the solid support in the sense of becoming a part of the solid support, and has the function of an anchoring part for the ligand comprising the at least one binding unit with at least one functional group.

Preferably, polyethyleneglycol (PEG) is used as spacer molecule, in a linear or branched configuration at the preferred average molecular weight of 400-10 000 Daltons and is represented by the formula H-(OCH₂CH₂)ₙ-OH,
wherein n is about 2-250. The flexibility of PEG chains allows a good access of toxin molecules within the three-dimensional structure of the ligand polymer.

Moreover, in another embodiment of the present invention the solid support may be provided with two or more spacers of the same or different kind, e.g. polyethyleneglycols, polypropyleneoxides, polyvinylalcohols, polyvinylamines, polyglycidoles, or polyethyleneimines, and derivatives thereof.

### The solid support

The solid support should provide variability in porosity and size exclusion characteristics. Also, it should provide a support for solid phase synthesis for polypeptide synthesis in the preferred embodiment. Different solid supports, i.e. polymers, that may be used in the present invention are described in EP 0 187 391, WO 99/17120, and the U.S. patent No. 4,908,405. Here, the solid support is a linear and/or branched biocompatible graft copolymer having a degree of cross-linking of 0.05-10% and is selected from the group consisting of polyvinylalcohols, polyhydroxystyrenes, polymers produced from chloromethylated polystyrenes and ethylene glycols; poly- or oligoethylene glycols of the formula H-(DCH₂CH₂)ₙ-OH, where n represents 2 to 250, polyacrylates or polymethacrylates functionalised with hydroxy groups; and derivatives thereof. In the present invention the above-mentioned degree of cross-linking may be up to 50%.

The solid support material is selected from a group consisting of, besides the above-mentioned, polystyrene, hydroxyalky-polystyrenes, hydroxyaryl-polystyrenes, hydroxyalky-aryl-polystyrenes, polyhydroxyalkylated polystyrenes, polyhydroxyarylated polystyrenes, isocyanatoalkyl-polystyrenes, isocyanatoaryl-polystyrenes, carboxyalkyl-polystyrenes, carboxyaryl-polystyrenes, aminoalkyl-polystyrenes, aminoaryl-polystyrenes, cross-linked polyethyleneglycols, polyacrylates, polymethacrylates, cellulose, silica, carbohydrates, latex, cyclo-olefine copolymers, glass, other suitable polymers or combinations thereof. The form of the solid support may be a bead, membrane, particle, e.g. a nanoparticle, net, woven and non-woven fabrics, fibre mat, tube, film, foil or combinations thereof, or cross-linked interpenetrating networks. In a preferred embodiment, the affinity matrix described above consists of cross-linked polystyrene onto which PEG as a spacer is grafted.

A preferred solid support material in the present invention is a bead, having a size sufficient to provide a highly perfusable and biocompatible support, e.g. polystyrene, preferably in the combination with PEG as a spacer to which lysine and/or arginine is attached, which should have no restrictions for hydrophilic or hydrophobic substances. A list of suitable commercially available beads is shown in Table 1.

**Table 1**

| |
|---|
| *Commercially* available activated *beads^{a}* |
| Toyo Pearl HW70EC^{®} (TosoHaas) |
| Toyo Pearl HW65EC^{®} (TosoHaas) |
| Toyo Pearl AF650M^{®} (TosoHaas) |
| |
| TentaGel^{®} (Rapp Polymer) |
| |
| Eupergit C250L^{®} (Röhm) |
| Eupergit 250^{®} (Röhm) |
| |
| Fractogel EMD Epoxy^{®} (M) (Merck) |
| Fractogel EMD Azlactone^{®} (S) (Merck) |
| |
| Poros EP^{®} (Perkin Elmer-Biosystems) |

| |
|---|
| ^{a} The commercially available activated beads above can be conditioned for immobilization of a ligand Perfusability of the polymer affinity matrix |

A hydrogel like structure is formed when the polymer affinity matrix is hydrated and, due to the hydration, swells. The swelling capacity is defined as the swelling due to hydration of the polymer from a dry state to form the hydrated and gel-like matrix. Such a swelling may be defined as an increase in weight per volume unit when hydrated and it may according to the invention be a factor of about 1.5 - 10 times, preferably 3 - 5 times, when comparing dry and hydrated forms of the polymer affinity matrix. This swelling capacity allows whole blood to perfuse completely through the matrix, still keeping the blood cells and numbers intact.

As stated above, in the preferred embodiment, the polymer affinity matrix as such generates a matrix with a defined cut off of about 1 x 10²-1 x 10⁶ Daltons allowing blood cells to perfuse and with almost no diffusional restriction for hydrophilic and/or hydrophobic substances.

### Method for removing a substance

The present invention also relates to a method for removing one or more substances from a fluid and/or reducing the amount or concentration thereof in said fluid with a view to preventing, eliminating or reducing undesired activation of components or processes in said fluid, comprising contacting the fluid with the polymer affinity matrix according to the present invention for a period of time sufficient to reduce the amount or concentration of and/or remove said substance(s). In a preferred embodiment using this method, the removal of a substance, e.g. an endotoxin from blood or any other body fluid, will thereby directly or indirectly prevent activation of e.g. blood cells by binding to the undesired substances listed under the definition of "substance(s)" above. Preferably this may be achieved by contacting the fluid with the polymer affinity matrix defined above for a period of up to 24 hours, most preferably from 1 s to 2 hours, giving a less activated or prevented activation of blood and its components.

In a preferred embodiment, the substance to be removed according to the method disclosed in the present invention is an endotoxin. The present invention also relates to removal of a defined blood cell type or population selected from leukocytes, e.g. T and B cells, monocytes, thrombocytes, granulocytes and/or neutrophiles. Also intended to be removed by the above described polymer matrix are components in an extra- or intracellular signalling transduction pathway selected from the group consisting of glucose and its degrading products, carbonyl compounds, inflammatory and proinflammatory proteins and/or proteins involved in thrombogenesis or in the complement cascade, bacteria derived constituents, endotoxins, cells, blood cells, bacteria and viruses, or pathogen-loaded blood cells, or at least parts or degradation products thereof, DNA or fragments thereof, or phosphate, by providing a ligand comprising at least one binding unit and having a structure which is complementary to the structure of a binding motif of the substance.

In this preferred embodiment, an endotoxin, e.g. LPS, is removed to < 50% over two hours during e.g. a static incubation or during perfusion of a solid phase column.

In this preferred embodiment, the endotoxin is removed during a defined time period of about 1 second to about and including 2 hours to an amount or concentration inactivating the blood or preventing activation of the blood, as measured according to an LAL assay, which is described below. Using the LAL assay is known in the art and will provide information about the endotoxin levels. The method according to the invention, which is fast and efficient, will yield levels of the endotoxin according to the LAL assay, within the above mentioned time period of 1 s-2 h, below the capacity of activating blood, or only activating blood cells and components to a minor degree.

### Method for producing a polymer affinity matrix

The method for producing a polymer affinity matrix according to the present invention comprises the following steps:
a) attaching the spacer to the solid support to obtain a first complex, and
b) attaching to said first complex the ligand containing said at least one binding unit with at least one functional group;
   or
c) attaching a spacer to the ligand containing said at least one binding unit with at least one functional group to obtain a second complex, and
d) attaching the solid support to said second complex;
   or
e) attaching the spacer to the solid support to obtain a first complex, and
f) solid phase synthesis of the ligand on the spacer bound to the solid support, or
g) building up or synthesizing the spacer from monomers directly on the solid support by grafting, and
h) attaching to said first complex the ligand containing said at least one binding unit with at least one functional group,
   or
i) building up or synthesizing the spacer from monomers directly on the solid support by grafting, and
k) solid phase synthesis of the ligand on the spacer bound to the solid support,
wherein information about the three-dimensional structure, presence of charges and hydrophobic/hydrophilic regions of the binding motif on the substance(s) to bind is collected from X-ray crystallography, protein modelling or hydrophobicity and hydrophilicity calculations and the ligand containing the binding unit is made complementary as regards charge and/or hydrophilicity/hydrophobicity to the binding motif of said substance(s).

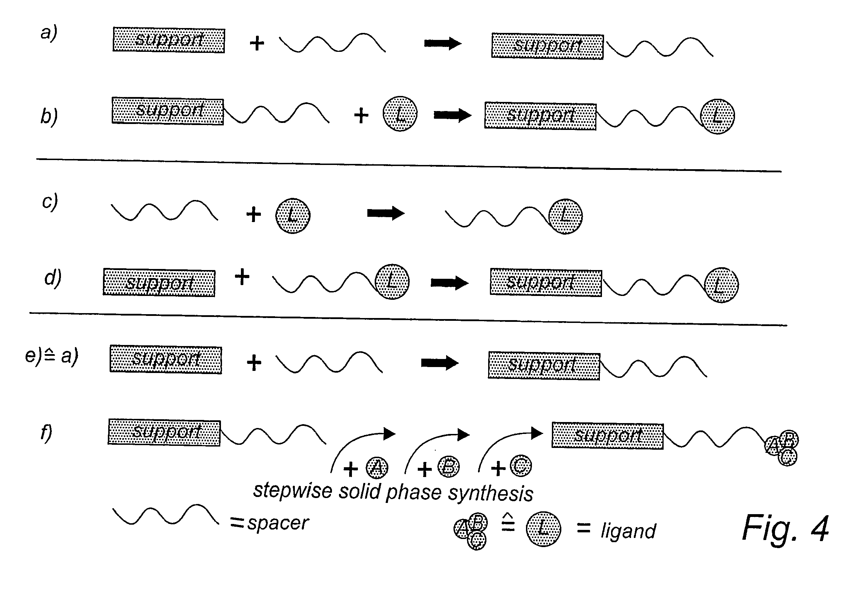

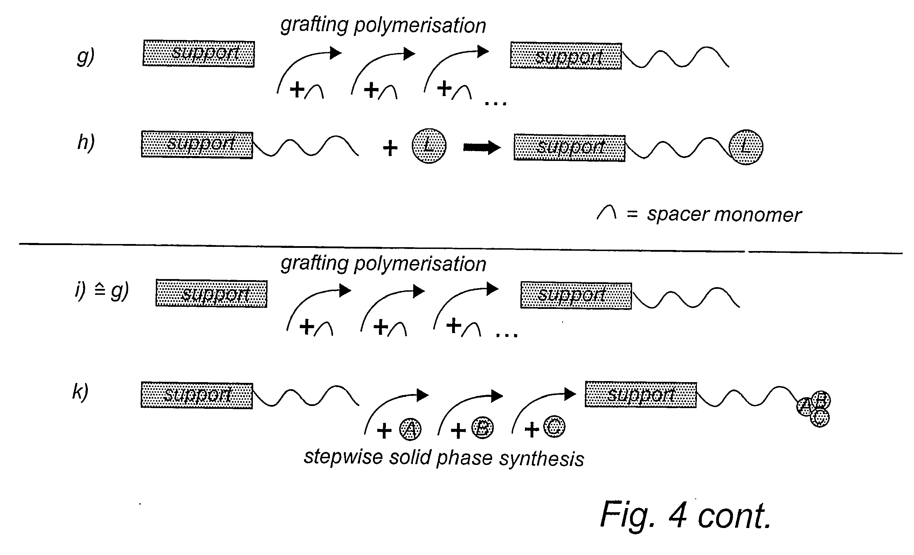

The method described above also includes in one embodiment the production of a matrix with a spacer molecule comprising a ligand immobilised on a solid support according to any of the following ways as shown more in detail in Fig. 4;
for a) and b) above: activation of the solid support, coupling of the spacer molecule to the solid support, synthesis of the ligand containing the binding unit, and site specific coupling of the ligand to the spacer molecule, or
for c) and d) above: synthesis of the ligand containing the binding unit, coupling of the spacer molecule to the ligand, activation of the solid support, and site specific coupling of the spacer-ligand complex to the activated solid support, or,
for e) and f) above: activation of the solid support, coupling of the spacer molecule to the activated solid support, and solid phase synthesis of the ligand containing binding unit on this support.

The method containing steps a) - f) described above also includes in a second embodiment the specific steps of, for a) and b), activation of the spacer, coupling of the activated spacer to the solid support, and coupling the ligand to said activated spacer, or,
for c) and d), synthesis of the ligand, activation of the spacer, site specific coupling of the ligand to the activated spacer molecule and coupling of the spacer-ligand complex to the solid support, or,
for e) and f), activation of the spacer, coupling of the activated spacer to the solid support and solid synthesis of the ligand on the spacer bound to the solid support.

As stated above, preferred by the present invention is the method above, wherein the solid support, the spacer and the ligand are immobilised by activation of a solid support, coupling of the spacer molecule, and solid phase synthesis of the ligand containing the binding unit directly on the solid support.

### Use of the polymer affinity matrix

The present invention also relates to the use of a polymer affinity matrix according to the present invention, preferably for use for removal of one or more substances, preferably endotoxins, from a fluid, or decreasing the amount or concentration thereof in said fluid, preferably a body fluid or a therapeutic fluid, most preferably blood, in particular for the production of less activated blood or prevention of undesired activation of components or processes in blood. The polymer affinity matrix is preferably used as a part in an extracorporeal blood purification process or in contact with blood or a blood stream, e.g. as an implant in the body to contact blood or any body fluid, e.g. the vascular system, blood vessels or in the peritoneal cavity.

### Kit comprising the polymer affinity matrix

In one embodiment the present invention refers to a kit for removing one or more substances from a fluid and/or decreasing the amount of or concentration thereof in said fluid with a view to preventing, eliminating, or reducing undesired activation of components in said fluid, said kit comprising a polymer affinity matrix according to the present invention, sample tubes, and a device for extra- and/or intracorporeal treatment of said fluid, preferably blood or serum.

### Conclusion

The present invention provides means for the removal of substances from a fluid in a highly efficient and time-saving way. This is achieved by the use of a polymer affinity matrix according to the invention that is highly biocompatible and allows for whole blood to flow through due to good swelling capacity. The efficient means are also due to the generation of a ligand comprising a binding unit complementary to the binding motif of the substance to be removed. Therefore, the present invention may be applied in e.g. extracorporeal blood treatment such as dialysis and transfusion medicine for therapeutic applications, stem cell therapy and/or therapeutic cell therapy, diagnostic applications and also in biotechnology, bioengineering, gene technology, food chemistry and water preparation and/or purification.

The present invention also concerns the use of a polymer matrix for the production of a polymer affinity matrix for removal of one or more substances from a fluid or decreasing the amount or concentration thereof in said fluid, wherein the specific affinity is dependent on any ligand applied on the polymer matrix, wherein the polymer matrix includes a solid support and a spacer, wherein the solid support is made of a material selected from the group consisting of polystyrene, polyvinyl alcohols, polyhydroxystyrenes, polymers produced from chloromethylated polystyrenes or polyacrylates, polymethacrylates functionalised with hydroxy groups, hydroxyalkyl-polystyrenes, hydroxyaryl-polystyrenes, hydroxyalkyl-aryl-polystyrenes, polyhydroxyalkylated polystyrenes, polyhydroxyarylated polystyrenes, isocyanatoalkyl-polystyrenes, isocyanatoaryl-polystyrenes, carboxyalkyl-polystyrenes, carboxyaryl-polystyrenes, aminoalkyl-polystyrenes, aminoaryl-polystyrenes, polymethacrylates, cross-linked polyethyleneglycols, cellulose, silica, carbohydrates, latex, cyclo-olefine copolymers, glass or combinations thereof, preferably a cross-linked polystyrene, and wherein the spacer is selected from the group consisting of poly- or oligoethylene glycols of the formula H-(OCH₂CH₂)ₙ-OH, wherein n represents 2-250.

In a preferred embodiment of the invention the solid support has the form of a bead, gel, membrane, particle, net, woven or non-woven fabric, fibre mat, tube, film, foil or combinations thereof or cross-linked interpenetrating networks.

In another preferred embodiment of the invention the spacer is a polyethylene glycol (PEG) in a linear and/or branched configuration and has an average molecular weight of 400-10 000 Daltons, or derivatives thereof.

In another preferred embodiment of the invention the polymer matrix has a swelling capacity enough to allow perfusion of plasma or whole blood.

In another preferred embodiment of the invention the swelling capacity of the polymer matrix is about 1.5-20 fold, preferably 2-6 fold, from a dry state to the hydrated form.

In another preferred embodiment of the invention the polymer matrix has the form of geltype beads.

In another preferred embodiment of the invention said fluid is an aqueous or organic solution, a body fluid, preferably blood, therapeutic fluids, fluids for life science applications, preferably buffer solutions, infusion fluids or dialysis fluids in biological, diagnostic or biotechnological application, blood products obtained from healthy donors, such as plasma, platelet concentrates, erythrocyte concentrates, preferably oxygen carriers, modified hemoglobin solutions and artificial hemoglobulin solutions, fluids for nutrition and fluids for industrial use.

In another preferred embodiment of the invention the polymer matrix has a cut-off value of from about 1 x 10² to about 1 x 10⁶ Daltons and binds hydrophobic and/or hydrophilic substances.

In another preferred embodiment of the invention the solid support is a cross-linked polystyrene, the spacer is a polyethylene glycol.

The rational development of specific binding structure applicable in extracorporeal blood treatment requires: (i) a flexible technology for building up ligands and (ii) basic requirements on biocompatibility, toxicology and processability. Solid phase peptide synthesis is applied to obtain well defined ligand structures assembled on biocompatible polymer substances, which is, according to one of the preferred embodiments, polystyrene-polyethylene glycol grafted copolymers specific for biologically substances.

By systematic variation of the geometrical structure of ligand motifs we could demonstrate that an increase in ligand affinity by a factor of 10 to 100 relative to the molar concentration of the building blocks. These investigations were carried out in human serum, i.e. in the presence of competitive proteins.

The assessment of blood compatibility was done by in vitro assays in human plasma and/or whole blood. The PS-PEG base material with or without the ligand is biocompatible with respect to complement activation, contact phase activation, cytotoxicity and granulocyte activation.

The base polymer structure shows an advantageous biocompatibility profile especially for extracorporeal application. The applied technology for ligand synthesis allows the processing of specific polymer materials without generation of toxic residuals.

### EXAMPLES

### Example 1

### Design of the ligand containing a binding unit for LPS

This example describes, without limiting the invention, the design of a ligand containing a binding unit for the removal of LPS.

### Principle

An efficient removal of toxins, e.g. LPS, requires a ligand with a binding unit that is complementary to the binding motif of the substance to be removed. This includes a three-dimensional aspect of the substance to be removed and a precise arrangement of molecules to optimise a biospecific recognition with characteristics like complementary charges, hydrophobicity and hydrophilicity. This, together with appropriate distances of the aforementioned characteristics, will complete the ligand with its binding unit. Also, the total three-dimensional presentation of such a ligand within a polymer matrix should be optimal in space for a high perfusion, ligand presentation and flexibility of the ligand. Still, a high biocompatibility is a prerequisite for in or ex vivo blood purification in patients. In the described matrix, the accessibility is comparable to or even identical to a non-solid phase aqueous solution. The structure of LPS is shown in Fig. 1. The suggested complementary binding to LPS is shown in Figs 3A-D, wherein the phosphate groups and hydrophobic tail of LPS are considered for the formation of a complementary binding motif. The suggested structure is shown for Arg₄ and Arg₈ in Fig. 2 as well as in a three-dimensional format in Fig. 11. The Arg₈ shows half of the arginine positions in the left handed Figure and half of the arginine positions in the right hand Fig. 11B. Also, the link to the spacer, here to PEG, is shown.

### Molecular simulation

Molecular simulations were further applied to identify and verify the three-dimensional geometric and chemical structure of the described binding site. The simulations were performed on a Silicon Graphics Octane2 Workstation, using the Insight II-Package (Molecular Simulations Inc. (MSI), San Diego, CA) and the optimisation algorithms included herein, e.g. AMBER-forcefields. The results of the tree-dimensional arrangements for Arg₄ and Arg₈ are shown in Figs 11A and B, respectively. Here, the illustration shows the principle of the three-dimensional terminal part of the matrix, without the PEG spacer or solid support.

### Synthesis of the ligand

The ligand with its binding unit is synthesised according to Fig. 4, wherein three different way are illustrated. A preferred method of the three is solid phase synthesis directly onto the PEG spacer attached to the solid support. The binding unit is used herein in further examples.

### Example 2

### Comparison of branched and linear ligands

This example describes the adsorption of the endotoxin LPS from human plasma. The example also shows a comparison between linear and branched ligands for the adsorption of the endotoxin.

### Principle

Beads are incubated with heparinised human plasma for a time period of two hours. The endotoxin levels are determined after two hours of incubation using an LAL assay.

### Material

The following beads are used:

| | |
|---|---|
| PS-PEG-LBP 94-108 | endotoxin-binding sequence from LBP, linear |
| PS-PEG-BPI 85-99 | endotoxin-binding sequence from BPI, linear |
| PS-PEG-Arg₈ | 8-fold branched containing arginine |
| PS-PEG-NH-Ac | acetylated base-material as control |
| No beads | Control |

### Procedure

Incubation of beads in heparinised plasma is carried out at 37 °C. Samples are slowly agitated and beads are removed by centrifugation after an incubation period of two hours.

### Analysis

An LAL assay is performed to quantitate the levels of endotoxins after the incubation with beads. The assay is performed by an LAL induced chromogenic substance reaction (Chromogenics, Mölndal, Sweden). Levels are calculated according to a standard curve obtained with defined concentrations of endotoxin in heparinised human plasma.

### Results

Using the LAL assay the following endotoxin concentrations were found after a two-hour incubation period.

| | Endotoxin (IU)/ml |
|---|---|
| PS-PEG-LBP 94-108 | 10 |
| PS-PEG-BPI 85-99 | 9 |
| PS-PEG-Arg₈ | 2 |
| PS-PEG-NH-Ac | 10 |
| No beads | 8 |
| Starting value | 10 |

### Discussion

This experiment shows that the endotoxin levels could be decreased 5-fold by using the branched, three-dimensional matrix onto the PS-PEG-beads. Beads with a linear ligand were not as efficient and endotoxin levels in those samples were still at, or near, starting levels of the endotoxin.

### Example 3

### Kinetics of endotoxin binding

This example shows the kinetics of endotoxin binding when beads with a three-dimensional matrix, optimised for LPS binding, are incubated with plasma and analysed at different points of time.

### Principle

The kinetics of absorption is dependent on the structure and the degree of cross-linking of the polymer matrix. Beads are incubated with heparinised human plasma. After 1, 10, and 120 minutes samples are withdrawn. The endotoxin levels are determined using an LAL assay.

### Material

The following beads are used:

| | |
|---|---|
| PS-PEG-Arg₈ | 8-fold branched containing arginine |
| PS-PEG-Arg₄ | 4-fold branched containing arginine |
| PS-PEG-Arg | linear containing arginine |
| PS-PEG-NH-Ac | acetylated base material (-Ref) |

### Procedure

Incubation of beads in heparinised plasma is carried out at 37° C. Samples are slowly agitated and test samples are withdrawn after an incubation period of 1, 10, 120 minutes. Beads are removed by centrifugation.

### Analysis

An LAL assay is performed for the quantification of endotoxin levels as in Example 2.

### Results

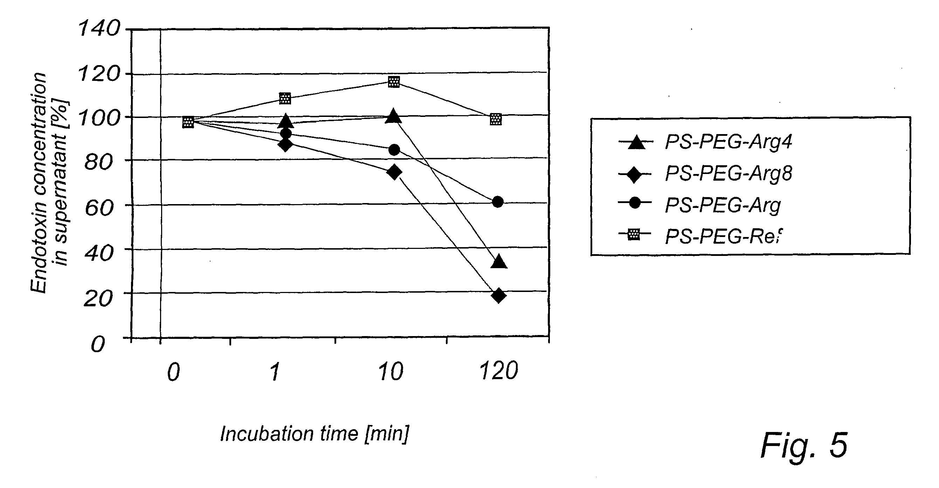

Fig. 5 shows the results of the endotoxin levels measured at different points of time. The Figure shows that a time period of two hours is needed for efficient removal, i.e. yielding 20-30% endotoxin left of the starting levels in the samples. The linear ligand containing arginine (PS-PEG-Arg) is only removing half the amount of endotoxins, i.e. a level of 60% left after 120 minutes. Similarly, where the reference beads (PS-PEG-NH-Ac) were added, the amount of endotoxin after incubation with the beads was still unchanged, i.e. at starting values, after 120 minutes.

### Example 4

### Influence of the terminal amino acid and branching of the polymer

This experiment shows the influence of the terminal amino acid and (arginine according to the present invention vs lysine) and the influence of branching, non-branched v.s. 4-fold v.s. 8-fold branched, of the ligand.

### Principle

Beads are incubated with heparinised human plasma with or without endotoxin. The endotoxin levels are determined using an LAL assay after two hours.

### Material

The following beads are used:

| | |
|---|---|
| PS-PEG-Arg₈ | 8-fold branched containing arginine |
| PS-PEG-Lys₈ | 8-fold branched containing lysine |
| PS-PEG-Arg₄ | 4-fold branched containing arginine |
| PS-PEG-Lys₄ | 4-fold branched containing lysine |
| PS-PEG-NH-Ac | acetylated base material |
| PS-PEG-Arg | linear containing arginine |
| No beads | Control |

### Analysis

An LAL assay is performed as in Example 2.

### Results

| | Endotoxin (IU/ml) |
|---|---|
| PS-PEG-Arg₈ | 0.4 |
| PS-PEG-Lys₈ | 1.8 |
| PS-PEG-Arg₄ | 0.01 |
| PS-PEG-Lys₄ | 2.7 |
| PS-PEG-NH-Ac | 7.8 |
| PS-PEG-Arg | 3.5 |
| No beads | 11 |
| Before incubation, i.e. | |
| starting values | 13 |

### Discussion

The results show that a branched ligand is more efficient than linear forms, and that arginine is several-folded (4-folded and 200-folded for Arg₈ and Arg₄ respectively) more efficient than lysine in the removal of endotoxins. Also, the PS-PEG-Arg₄ is the most efficient in the removal of endotoxins after two hours.

### Example 5

### Reduction of endotoxin dependent IL6 induction in CD14⁺ monocytes

This example describes the reduction of endotoxin dependent IL6 induction in human monocytes by the use of PS-PEG-Arg₈.

### Principle

In response to LPS, monocytes start to transcribe and express IL6. The levels of upregulated IL6 can already be measured after 4 h by intracellular flow cytometry analysis (FACS).

### Material

Human mononuclear cells from whole blood are used. PS-PEG-Arg₈ is used for the removal of endotoxin and PS-PEG-NH-Ac is included as a control. Lipopolysaccaride (E. coli strain 055:B5 from Biowittaker Co.) is used for stimulation of the MNC *in vitro.* FACS analysis is performed using a FACSCAN Calibur (Beckton Dickinson^{®})

### Procedure

Whole blood is incubated at 37° C (5% CO₂) for 30 minutes with 10 or 30 IU/ml LPS. Samples are incubated in parallel with and without PS-PEG-Arg₈ beads, as well as control beads PS-PEG-NH-Ac. The cells are fixed in PermFix (Beckton Dickinson^{®}) and a double staining performed with anti-CD14-FITC and anti-IL6-PE. 20 000 cells are counted per cell sample for FACS analysis. Monocytes defined as CD14⁺ cells normally constitute about 5% of the total cell population.

### Analysis

Monocytes are defined as CD14⁺ cells in the cell suspension. Intracellular IL6 (icIL6) is calibrated to an internal standard and quantitated in the CD14⁺ cell population using FACS analysis after the incubation with 10 or 30 IU/ml LPS for 30 minutes. Calculations are performed using CellQuest Software Package (Beckton Dickinson^{®}).

### Results

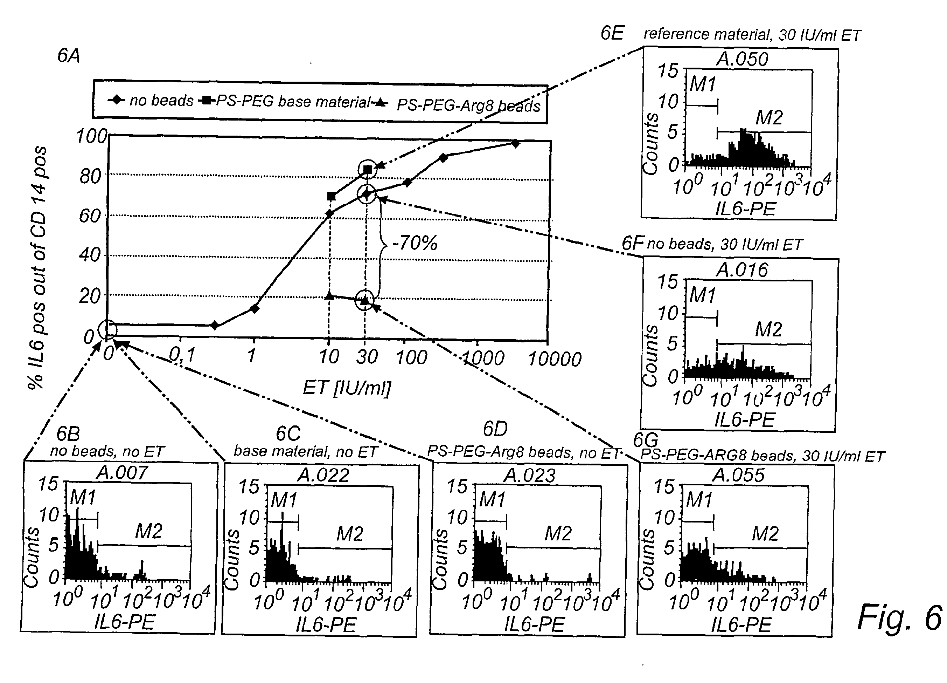

Using the FACS data and calculations performed therefrom, analysis by a skilled man in the art of the CD14⁺ monocyte population generates the following data displayed in Fig. 6. Shown in the graph is a 70% reduction of the endotoxin levels when using PS-PEG-Arg₈ beads compared to the control beads (PS-PEG-NH-Ac) and samples with no beads included. The intracellular levels of IL6 are shown in histograms. On the right hand side, a decrease can be seen in IL6 levels after incubation with PS-PEG-Arg₈ beads. The lower left row shows the absence of intracellular IL6 in fresh cells.

### Discussion

This experiment shows a rapid and complete inhibition of LPS stimulation in a human CD14⁺ monocyte population measured by an inhibition of the LPS dependent IL6 upregulation in monocytes.

### Example 6

### Cell counts after whole blood perfusion

This example describes, without limiting the invention, the permeabilisation of blood cells after perfusion through the polymer affinity matrix on beads.

### Principle

Blood purification requires no damage to or activation of cells. Mechanical damage, especially of erythrocytes, could lead to haemolysis and subsequent life-threatening complications. A hydrogel like polymer matrix according to the invention can surprisingly be perfused by whole blood cells.

### Material

A highly swellable polymer matrix, PS-PEG-Arg₈ beads, are used. Columns, ID 20 mm, are filled with 1g of the matrix, absorbing 4-5 ml water and subsequently prerinsed with physiological saline solution before use. The columns are then used for perfusion of whole blood. Procedure

Fresh whole blood with citrate and LPS (30 IU/ml) is perfused through columns using sterile equipment in a laminar flow. LPS-spiked whole blood is perfused at a flow of 5 ml/min at 37 °C. Blood samples were drawn before and after perfusion and analysed. Cells analysed are red blood cells (RBC), hematocrit (HTC) and free hemoglobin, white blood cell (WBC), platelets, and thrombocyte numbers (TRC).

Cell number is counted pre- and post column perfusion by a Coulter Counter^{®} (Becton Dickinson).

Free hemoglobin is measured as a total hemoglobin level after a complete lysis of erythrocytes followed by a photometric quantification at 405 nm. Integrity of erythrocytes after whole blood perfusion is shown by measurements of free hemoglobin in plasma by a photometric quantification at 405 nm.

### Results and discussion

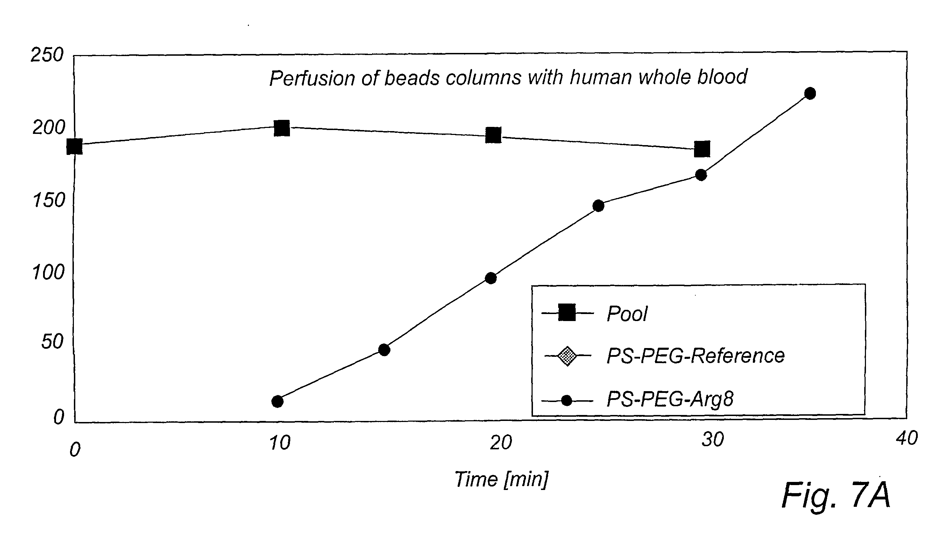

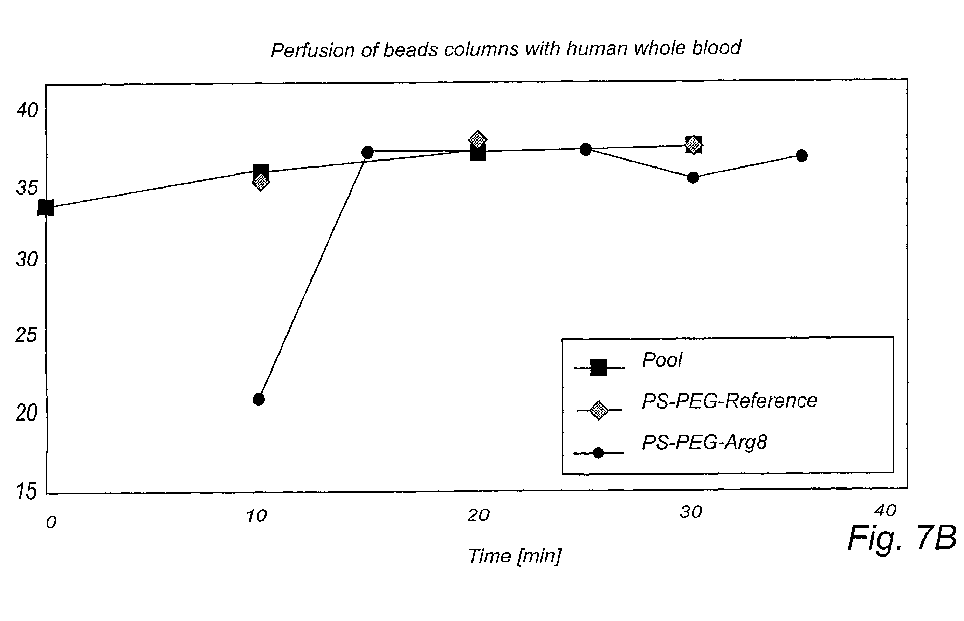

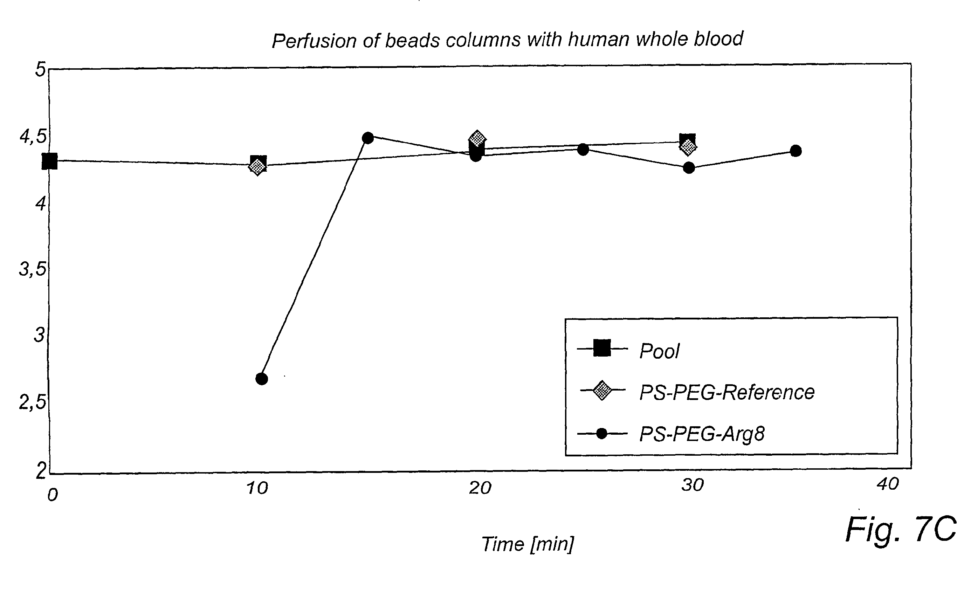

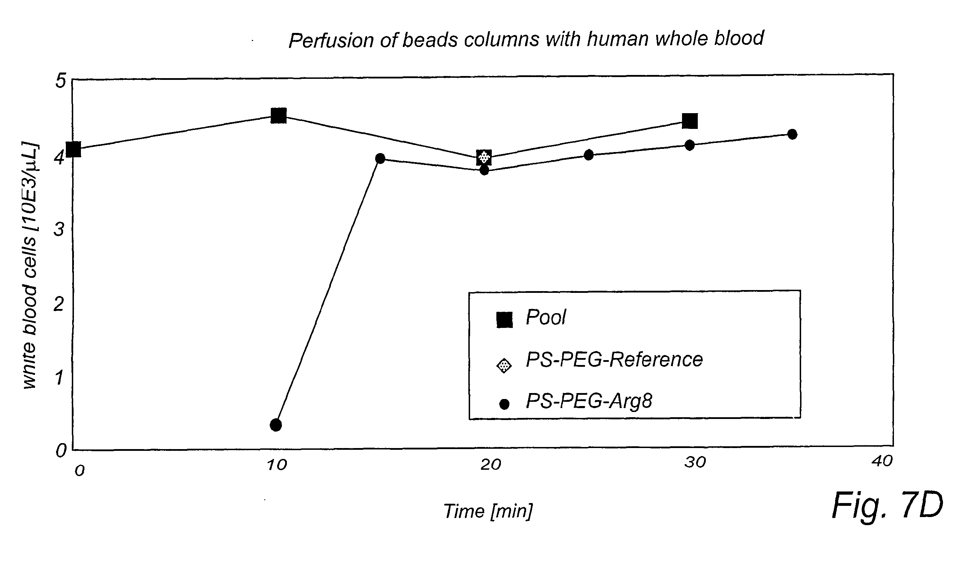

Cell numbers and HCT are measured and the results are shown in Figs 7A (thrombocytes), 7B (HCT), 7C (RBC), and 7D (WBC). In addition, no increase in free hemoglobin is found, i.e. no haemolysis is induced (data not shown). Data from this experiment shows that, after the initial transient retention time due to an initial dilution effect, the cell number is stabilising to pre-column values. The data shows a permeabilisation of the matrix material of about 100%, since the pre-column cell count is the same as the post-column cell count. Also, the cells keep intact, as measured by viable cell count and red blood cell lysis.

### Example 7

### Biocompatibility features

This example describes parts of the biocompatibility profile of the PS-PEG-Arg₈ matrix.

### Principle

Biocompatibility or non-compatibility is herein measured as complement activation, elastase release, and trombogenicity by thrombin-antithrombin III complex formation.

### Material

As in Example 6, cellulosic material is used as a reference.

### Method

Elastase is measured in plasma from whole blood perfusion using a specific enzyme linked immunosorbent assay (ELISA; Diagnostic Product Co.).

Complement activation is measured by quantitation of terminal complement complex (TCC protein) is measured according to Deppisch et al. (Kidney Int. 37:696-706).

TAT, the degree of thrombogenicity, is measured according to Deppisch et al. (Neuphrol. Dial. Transplant Suppl. 3:17-23, 1994).

### Results

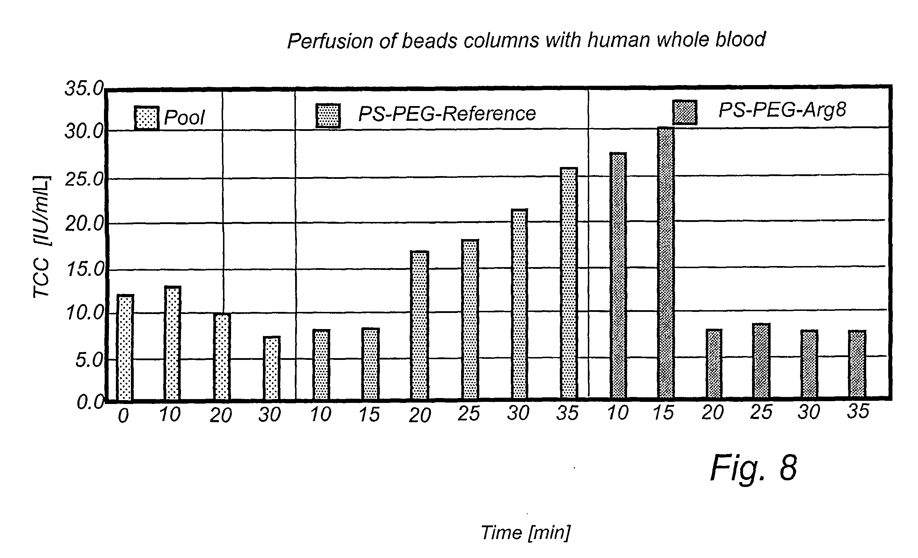

In Fig. 8, the formation of TCC is shown over time. Reference beads, PS-PEG-NH-Ac (TG-base or -ref material) shows a 4-5 fold increase in TCC formation. After an initial rise over 10-15 min for PS-PEG-Arg₈ the TCC formation stabilises at pre-perfusion levels.

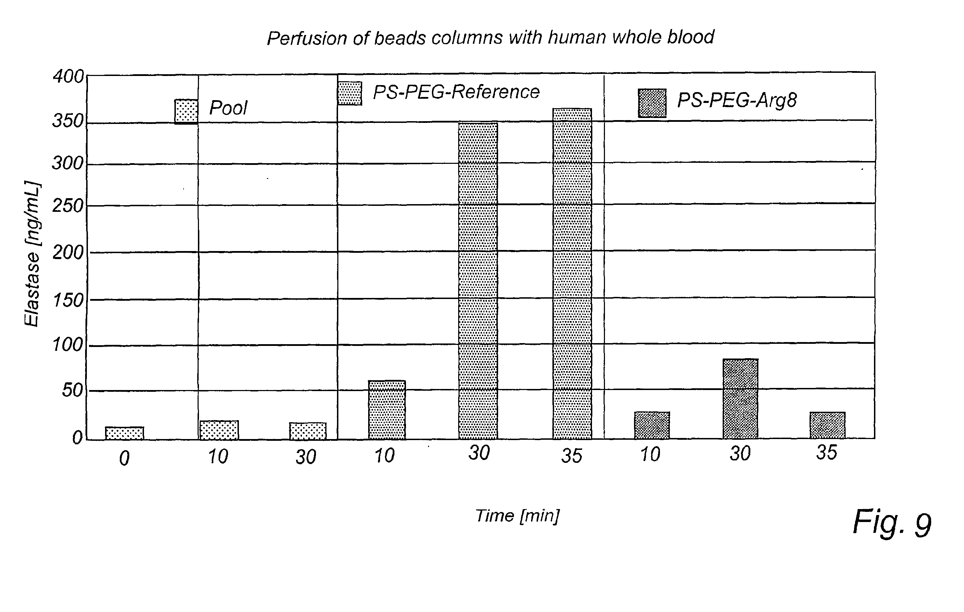

In Fig. 9, the level of elastase is measured over time. Levels are not changing compared to pre-column values. Measurements over 35 minutes are shown. For the reference material the elastase levels are increasing 8-9 fold over 35 minutes.

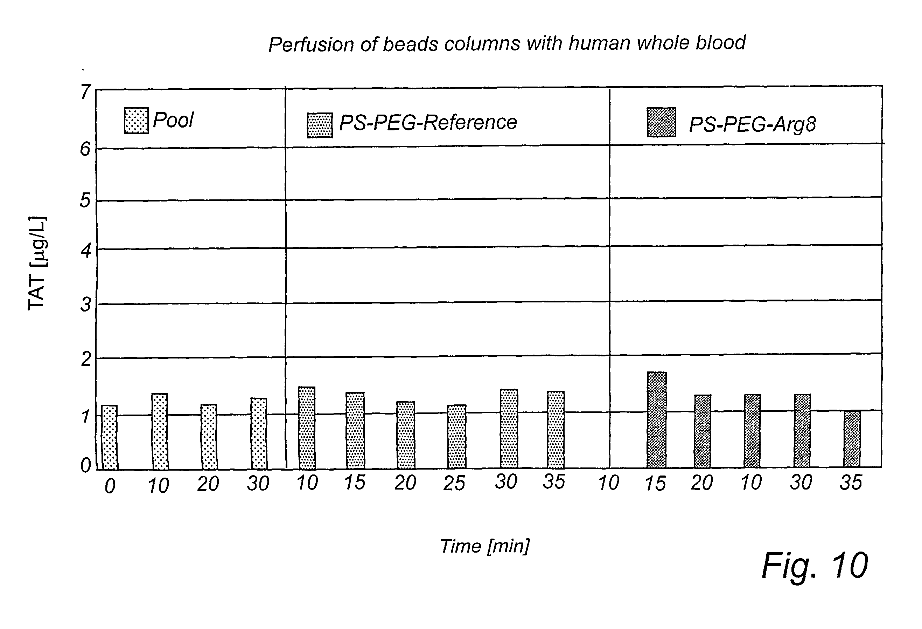

In Fig. 10, the formation of TAT is shown. No increase in TAT formation is detected.

### Discussion

Biocompatibility features are important in ex vivo and in vivo blood treatment, e.g. dialysis. The described matrix shows no activation of the complement system, no activation of the coagulation system and no elastase release compared to the reference material included, i.e. a high biocompatibility. This, together with the high perfusion capacity of whole blood as shown in Example 6, shows that the polymer affinity matrix according to the invention is highly suitable for extracorporeal treatment of blood, whole blood included.

### Example 8

### Further endotoxin adsorption experiments

Endotoxin adsorption experiments have been performed in order to show the importance of finding a specific geometrically defined arrangement of functional groups (i.e. arginine residues) which build up a ligand for endotoxin binding. It is shown that the endotoxin binding does not just depend on the amount of arginine (i.e. the positive charges) but that the defined geometrical arrangement is more important.

### Procedure

Human serum (as a representative of human body fluids such as blood, plasma etc) was spiked with defined amounts of endotoxin (i.e. 3 IU/ml and 10 EU/ml) and incubated with 40 mg of PS-PEG-Beads containing either the Arg8 arrangement or the Arg4 arrangement as ligand. The two kinds of beads/ligand arrangements contain different amounts of arginine: Arg4 contains 0.72 mmol/g and Arg8 contains 1.89 mmol/g. The higher arginine content of the Arg8 beads is a consequence of the additional branching caused by the lysine bifurcations. After 30 min of incubation the free (i.e. not bound to the surface or matrix) concentration of endotoxin was measured (by LAL test) in the supernatant serum.

### Analysis

**Table 2 Results of the incubation experiment with human serum**

| spiked serum before incubation | | 0 | 3 | 10 | EU/ml |
|---|---|---|---|---|---|
| serum after 30 min incubation | no beads | 0 | 3 | 9.1 | EU/ml |
| | PS-PEG-Arg4 | 0 | 1.5 | 5.5 | EU/ml |
| | PS-PEG-Arg8 | 0 | 0.2 | 1.6 | EU/ml |

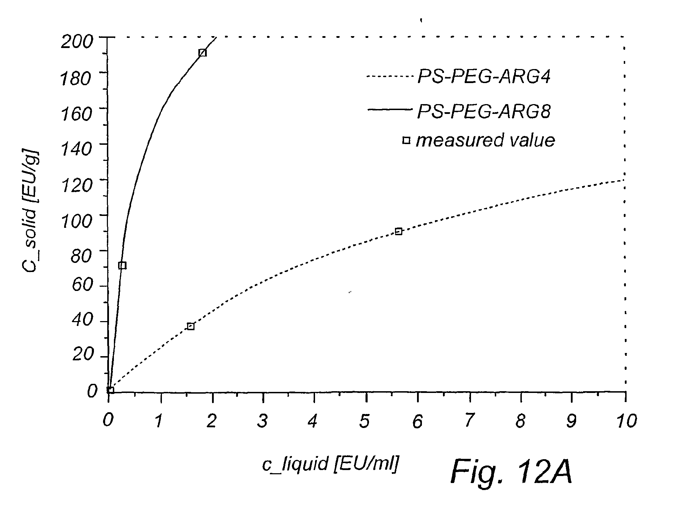

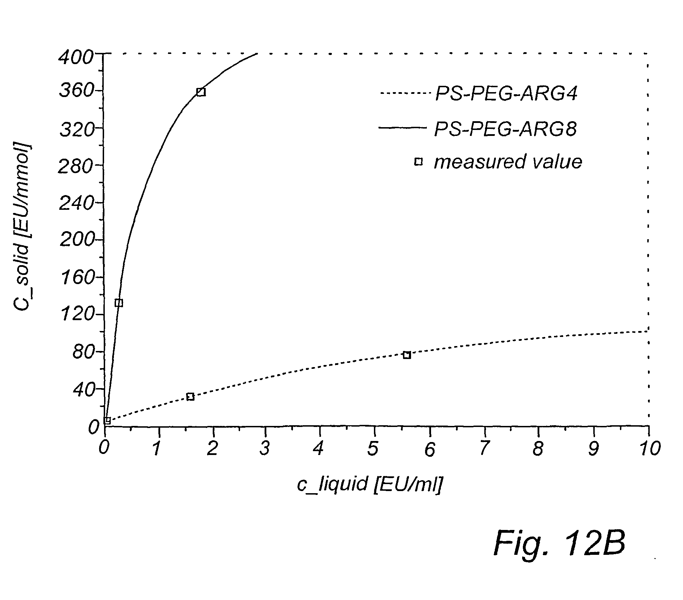

These data were analyzed by using the Langmuir approach which describes ligand-receptor interactions and equilibria at solid surfaces (e.g. protein adsorption) (Ref: JD Andrade: Principles of protein adsorption. in: JD Andrade (Ed.) Surface and Interfacial Aspects of Biomedical Polymers. Volume 2, Protein Adsorption, Plenum Press New York 1985, pp 1-80.). This analysis is done by plotting the amount of toxin (i.e. endotoxin) bound to the ligand-matrix (calculated from the difference between amount added and amount left in the supernatant after incubation) divided by the total number of ligands present at the matrix versus the equilibrium concentration of the toxin (i.e. the endotoxin concentration after incubation) in the supernatant. The plots are then fitted to the mathematical expression of the Langmuir isotherm, which gives the affinity or association constants for the different matrix-bound ligands towards the toxin. In Figs 12A and 12B examples of the Langmuir approach are shown.

Two kinds of Langmuir plots were performed, which differ by the way the total number of ligands present at the matrix is defined:
(1) neglecting the different arginine content of the beads, i.e. by just taking the mass of beads in grams as a measure of the total number of ligands (Fig. 12A); this approach is justified since in the practical application of the ligand-polymer-matrix, e.g. in an adsorber device to be perfused with a human body fluid such as blood or plasma in an extracorporeal circuit, it is to a certain extent limited by the total mass or volume of beads which has to be filled into the device in order to achieve a therapeutically effective reduction of the concentration of a toxin in the patient. The limitation is e.g. related to pressure drop across the device, non-specific effects on blood components (proteins and cells) or even storage space for the device.
(2) taking into account the different arginine contents of the beads, i.e. by taking the mass of beads in grams divided by the arginine load in mmol/gram as a measure of the total number of ligands (Fig. 12B) ; this approach is justified by the fact that the arginine content is a main cost factor in the manufacturing of the ligand-polymer-matrix; the result describes the efficiency of the ligands from an economical point of view.

The equilibrium parameters for the different Langmuir plots are shown in Table 3.

**Table 3: Equilibrium parameters taken from the fitted curves**

| | | PS-PEG-Arg8 | PS-PEG-Arg4 | unit |
|---|---|---|---|---|
| analysis 1 | affinity constant | 1.81 | 0.15 | ml/EU |
| | saturation concentration | 253.9 | 200.7 | EU/g |
| analysis 2 | affinity constant | 1.81 | 0.15 | ml/EU |
| | saturation concentration | 479.9 | 168.6 | EU/mmol |

### Results and discussion

The affinity constant describes and characterizes how strongly a certain ligand is able to bind a toxin, independently of the amount of ligand present. Surprisingly, the affinity constant of the Arg8 ligand was more than 10-fold higher (i.e. by a factor of 12.3) than the affinity constant of the Arg4 ligand.

The saturation concentration describes the maximum amount of toxin which can be bound by the respective ligand-matrix, assuming that unlimited amounts of toxins are available. Surprisingly the saturation concentration of the Arg8 ligand-matrix was more than 2-fold higher (i.e. by a factor of 2.8) than the saturation concentration of the Arg4 ligand-matrix. Since according to the Langmuir adsorption model the saturation concentration is completely independent of the affinity constant, this can be interpreted in a way that the specific geometrical arrangement of the Arg 8 ligand influences the accessibility of the ligand-matrix for the toxins, e.g. by influencing the morphology (e.g. crystal-like vs. fluidic) of the PEG-part of the ligand-matrix.

The following example demonstrates the importance and relevance of the improved affinity of the Arg8 ligand-matrix endotoxin with respect to the amount of matrix which is necessary to achieve a therapeutically relevant reduction in the toxin concentration: Endotoxin concentrations in the range of 0.1 to 1 EU/ml are found in septic patients (e.g. Nakamura et al. Renal Failure 2000; 22: 225-234). Assuming a plasma concentration of 0.3 EU/ml at the start of the treatment and a plasma concentration of 0.03 EU/ml after treatment and a plasma volume of 4000 ml, this means that 1080 EU endotoxin have to be bound to the ligand-matrix during treatment. The amount of matrix necessary to bind this amount of endotoxin can be calculated from the Langmuir expression using the affinity constants and the saturation concentrations for the respective ligands. The concentration at the end of the treatment is then the equilibrium concentration. Using the equilibrium data from Table 3 this calculation shows that for the Arg8 ligand-matrix only 83 g are necessary, whereas for the Arg4 ligand-matrix 1201 g are necessary to achieve the therapeutic goal. Since both ligand-matrixes have a similar density (mass per volume) this means that the volume and size of an adsorber device will be much smaller for the Arg8 ligand, which facilitates perfusion by plasma or blood.

## Claims

1. A polymer affinity matrix for binding one or more substances in a fluid for removing said substance(s) from the fluid and/or decreasing the amount or concentration thereof in said fluid with a view to preventing, eliminating, or reducing undesired activation of components or processes in said fluid, wherein said matrix comprises
a) a solid support
b) at least one spacer bound to the solid support, wherein the spacer is selected from the group consisting of poly- or oligoethylene glycols of the formula H-(OCH₂CH₂)ₙ-OH, wherein n represents 2-250, or polyvinylalcohols, polyvinylamines, polyolycidoles, polyethyleneimines, polypropyleneoxides, or derivatives thereof, and, coupled to each spacer,
c) a ligand having a defined three-dimensional structure which is complementary as regards charge and/or hydrophobicity/hydrophilicity to the three-dimensional structure of a binding motif of said substance(s), wherein the ligand is chosen from the group consisting of 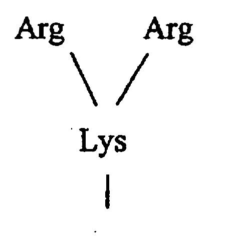 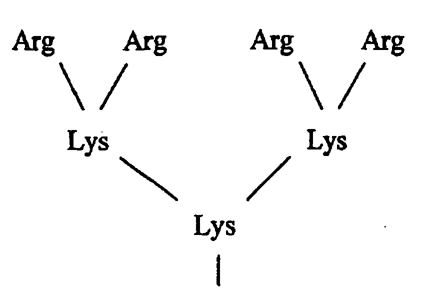 and

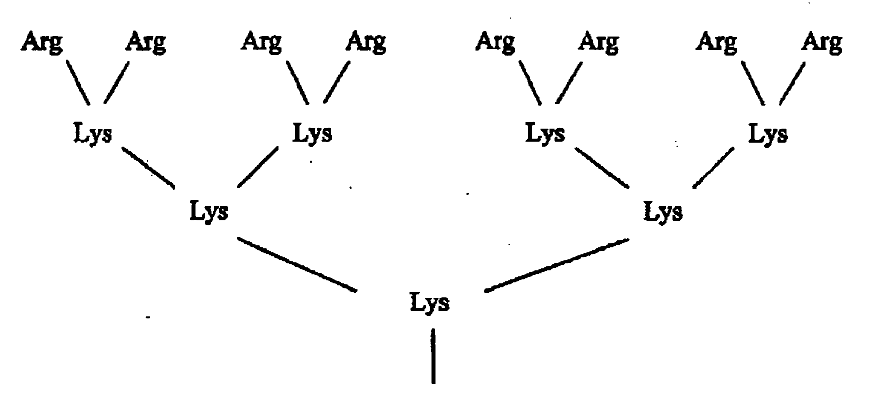

2. The polymer affinity matrix according to claim 1, wherein the spacer is a polyethylene glycol (PEG) in a linear and/or branched configuration and has an average molecular weight of 400-10 000 Daltons, or derivatives thereof.

3. The polymer affinity matrix according to claim 1 or 2, wherein the solid support is made of a material selected from the group consisting of polystyrene, polyvinyl alcohols, polyhydroxystyrenes, polymers produced from chloromethylated polystyrenes or polyacrylates, polymethacrylates functionalised with hydroxy groups, hydroxyalkyl-polystyrenes, hydroxyaryl-polystyrenes, hydroxyalkyl-aryl-polystyrenes, polyhydroxyalkylated polystyrenes, polyhydroxyarylated polystyrenes, isocyanatoalkyl-polystyrenes, isocyanatoaryl-polystyrenes, carboxyalkyl-polystyrenes, carboxyaryl-polystyrenes, aminoalkyl-polystyrenes, aminoaryl-polystyrenes, polymethacrylates, cross-linked polyethyleneglycols, cellulose, silica, carbohydrates, latex, cyclo-olefine copolymers, glass or combinations thereof, preferably a cross-linked polystyrene.

4. The polymer affinity matrix according to claim 3, wherein the solid support has the form of a bead, gel, membrane, particle, net, woven or non-woven fabric, fibre mat, tube, film, foil or combinations thereof or cross-linked interpenetrating networks, preferably a polystyrene-PEG bead.

5. The polymer matrix according to any one of the preceding claims, wherein said matrix is biocompatible and has a swelling capacity enough to allow perfusion of whole blood.

6. The polymer matrix according to claim 5, wherein the swelling capacity is about 1.5-20 fold, preferably 2-6 fold, from a dry state to the hydrated form.

7. The polymer affinity matrix according to any one of the preceding claims, wherein said matrix provides a three-dimensional complementary structure for binding at least one substance selected from the group consisting of bacteria or virus derived constituents, endotoxins, exotoxins, bacterial DNA or fragments thereof, oligonucleotides; cells, in particular endothelial cells, stem cells, and tumour cells; blood cells, in particular lymphocytes, thrombocytes, granulocytes, dendritic cells, and monocytes; prions, parasites, fungi, drugs after overdosing, pathogenic food additives, products from acute or chronic metabolic disturbances resulting from diabetes mellitus, liver disease, uraemia, kidney diseases or inflammation, heparin, bacteria and viruses, pathogen-loaded blood cells, or at least parts or degradation products thereof, DNA, phosphate, cytokines, growth factors, hormones, chemokines, uremic toxins, blood clotting proteins, procoagulatory proteins, inflammatory or proinflammatory proteins, macrophage migration inhibitory factor, soluble or cell surface bound proteins, soluble adhesion molecules, and glucose or degradation products thereof, pyrogens, bacterial exotoxins, products from Gram-positive bacteria, preferably lipoteichonic acid, in particular bacterial pyrogens, preferably endotoxins, in particular the lipid A component of lipopolysaccharides (LPS).

8. The polymer affinity matrix according to any one of the previous claims, wherein said matrix has a cut-off value of from about 1 x 10² to about 1 x 10⁶ Daltons and binds hydrophobic and/or hydrophilic substances.

9. The polymer affinity matrix according to any one of the preceding claims, wherein the fluid which the substance(s) is/are to be removed from or to be reduced in is an aqueous or organic solution, a body fluid, preferably blood, therapeutical fluids, fluids for life science applications, preferably buffer solutions, infusion fluids or dialysis fluids in biological, diagnostic or biotechnological applications, blood products obtained from healthy donors, such as plasma, platelet concentrates, erythrocyte concentrates which are used for transfusions, blood substitutes, preferably oxygen carriers, modified hemoglobin solutions and artificial hemoglobin solutions; fluids for nutrition and fluids for industrial use.

10. The polymer affinity matrix according to any one of the preceding claims, wherein the solid support is a cross-linked polystyrene, and the spacer is a polyethylene glycol.

11. A method for removing one or more substances from a fluid and/or reducing the amount or concentration thereof with a view to preventing, eliminating or reducing undesired activation of components or processes in said fluid, comprising contacting the fluid with the polymer affinity matrix as defined in any one of claims 1-10 for a period of time sufficient to reduce the amount or concentration and/or remove said substance(s), preferably up to 24 hours.

12. The method according to claim 11, wherein the period of time is from 1 s to 2 hours.

13. The method according to any one of claims 11 and 12, wherein the substance is an endotoxin and the fluid is blood, wherein the amount or concentration of endotoxin after being removed or reduced is below the capacity of activating components in blood or prevents activation of components or processes in blood.

14. Use of the polymer affinity matrix as defined in any one of claims 1-10 for removal of one or more substances, preferably endotoxins, from a fluid, or decreasing the amount or concentration thereof in said fluid, preferably a body fluid or a therapeutic fluid, most preferably blood or serum.

15. The use according to claim 14 for production of less activated blood or prevention of undesired activation of components or processes in blood.

16. The use according to claim 14 as a part of an extracorporeal blood purification process or as an implant in the body to contact blood or any body fluid, preferably in the vascular system, blood vessels or the peritoneal cavity.

17. The use according to claim 16 for production of less activated blood or prevention of undesired activation of components or processes in blood.

18. A kit for removing one or more substances from a fluid and/or decreasing the amount or concentration thereof in said fluid with a view to preventing, eliminating, or reducing undesired activation of components or processes in said fluid, said kit comprising a polymer affinity matrix as defined in any one of claims 1-10.

19. The kit according to claim 18, wherein it further comprises sample tubes, and a device for extra- and/or intracorporeal treatment of said fluid, preferably blood or serum.

20. A method for producing a polymer affinity matrix as defined in any one of claims 1-10, comprising
a) attaching the spacer to the solid support to obtain a first complex, and
b) attaching to said first complex the ligand containing said at least one binding unit with at least one functional group;
or
c) attaching the spacer to the ligand containing said at least one binding unit with at least one functional group to obtain a second complex, and
d) attaching the solid support to said second complex;
or
e) attaching the spacer to the solid support to obtain a first complex, and
f) solid phase synthesis of the ligand on the spacer bound to the solid support, or
g) building up or synthesizing the spacer from monomers directly on the solid support by grafting, and
h) attaching to said first complex the ligand containing said at least one binding unit with at least one functional group,
or
i) building up or synthesizing the spacer from monomers directly on the solid support by grafting, and
k) solid phase synthesis of the ligand on the spacer bound to the solid support,
wherein information about the three-dimensional structure, presence of charges and hydrophobic/hydrophilic regions of the binding motif on the substance(s) to bind is collected from X-ray crystallography, protein sequencing, protein modelling or hydrophobicity and hydrophiliccity calculations and the ligand containing the binding unit is made complementary as regards charge and/or hydrophilicity/hydrophobicity to the binding motif of said substance(s).

21. The method according to claim 20 comprising the steps of,
for a) and b), activation of the solid support, coupling of the spacer molecule on the solid support, synthesis of the ligand containing the binding unit, and site specific coupling of the ligand to the spacer molecule, or,
for c) and d), synthesis of the ligand containing the binding unit, coupling of the spacer molecule to the ligand, activation of the solid support, and site specific coupling of the spacer-ligand complex to the solid support, or,
for e) and f), activation of the solid support, coupling of the spacer molecule to the activated solid support, and solid phase synthesis of the ligand on the spacer bound to the support.

22. The method according to claim 21 comprising the steps of,
for a) and b), activation of the spacer, coupling of the activated spacer to the solid support, and coupling the ligand to said activated spacer, or,
for c) and d), synthesis of the ligand, activation of the spacer, site specific coupling of the ligand to the activated spacer molecule and coupling of the spacer-ligand complex to the solid support, or,
for e) and f), activation of the spacer, coupling of the activated spacer to the solid support and solid synthesis of the ligand on the spacer bound to the solid support.

## Patentansprüche

1. Polymeraffinitätsmatrix zum Binden einer oder mehrerer Substanzen in einem Fluid zum Entfernen der Substanz(en) aus dem Fluid und/oder zum Erniedrigen ihrer Menge oder Konzentration in dem Fluid im Hinblick auf das Verhindern, Eliminieren oder Verringern von unerwünschter Aktivierung von Komponenten oder Vorgängen in dem Fluid, worin die Matrix umfasst
a) einen festen Träger,
b) wenigstens eine an den festen Träger gebundene Abstandsgruppe, worin die Abstandsgruppe ausgewählt ist aus der Gruppe bestehend aus Poly- oder Oligoethylenglycolen der Formel H-(OCH₂CH₂)ₙ-OH, worin n 2 bis 250 bedeutet, oder Polyvinylalkoholen, Polyvinylarlinen, Polyglycidolen, Polyethyleniminen, Polypropylenoxiden oder Derivaten davon, und, gekuppelt an jede Abstandsgruppe,
c) einen Ligand mit einer definierten dreidimensionalen Struktur, die, was die Ladung und/oder Hydrophobie/Hydrophilie betrifft, zu der dreidimensionalen Struktur eines bindenden Prinzips der Substanz(en) komplementär ist, worin der Ligand ausgewählt ist aus der Gruppe bestehend aus 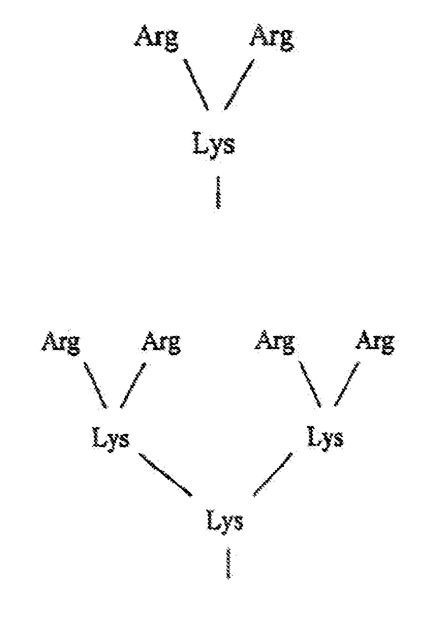 und

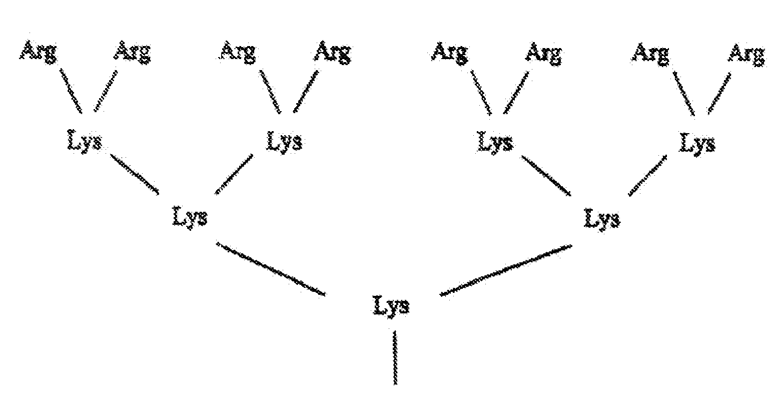

2. Polymeraffinitätsmatrix gemäß Anspruch 1, worin die Abstandsgruppe ein Polyethylenglycol (PEG) in einer linearen und/oder verzweigten Konfiguration ist und ein mittleres Molekulargewicht von 400 bis 10000 Dalton hat, oder Derivate davon.

3. Polymeraffinitätsmatrix gemäß Anspruch 1 oder 2, worin der feste Träger aus einem Material hergestellt ist, ausgewählt aus der Gruppe bestehend aus Polystyrol, Polyvinylalkoholen, Polyhydroxystyrolen, aus chlormethylierten Polystyrolen oder Polyacrylaten hergestellten Polymeren, mit Hydroxygruppen funktionalisierten Polymethacrylaten, Hydroxyalkylpolystyrolen, Hydroxyarylpolystyrolen, Hydroxyalkylarylpolystyrolen, polyhydroxyalkylierten Polystyrolen, polyhydroxyarylierten Polystyrolen, Isocyanatoalkylpolystyrolen, Isocyanatoarylpolystyrolen, Carboxyalkylpolystyrolen, Carboxyarylpolystyrolen, Aminoalkylpolystyrolen, Aminoarylpolystyrolen, Polymethacrylaten, vernetzten Polyethylenglycolen, Cellulose, Siliciumdioxid, Kohlenhydraten, Latex, Cycloolefin-Copolymeren, Glas oder Kombinationen davon, bevorzugt einem vernetzten Polystyrol.

4. Polymeraffinitätsmatrix gemäß Anspruch 3, worin der feste Träger die Form einer Perle, eines Gels, einer Membran, eines Teilchens, eines Netzes, eines Gewebes oder eines Faservlieses, einer Fasermatte, eines Rohrs, eines Films, einer Folie oder Kombinationen davon oder vernetzter interpenetrierender Netzwerke, bevorzugt einer Polystyrol-PEG-Perle, hat.

5. Polymermatrix gemäß einem der vorhergehenden Ansprüche, worin die Matrix biokompatibel ist und eine Quellfähigkeit hat, die hoch genug ist, um eine Durchströmung von Vollblut zu erlauben.

6. Polymermatrix gemäß Anspruch 5, worin die Quellfähigkeit das etwa 1, 5- bis 20-fache, bevorzugt das 2- bis 6-fache, vom trockenen Zustand zu der hydratisierten Form beträgt

7. Polymeraffinitätsmatrix gemäß einem der vorhergehenden Ansprüche, worin die Matrix eine dreidimensionale komplementäre Struktur zum Binden an wenigstens eine Substanz bereitstellt, ausgewählt aus der Gruppe bestehend aus von Bakterien oder Viren abgeleiteten Bestandteilen, Endotoxinen, Exotoxinen, bakterieller DNA oder Fragmenten davon, Oligonukleotiden; Zellen, insbesondere Endothelzellen, Stammzellen und Tumorzellen; Blutzellen, insbesondere Lymphozyten, Thrombozyten, Granulozyten, dendritischen Zellen und Monozyten, Prionen, Parasiten, Pilzen, Wirkstoffen nach Überdosierung, pathogenen Nahrungsmittelzusätzen, Produkten aus akuten oder chronischen Stoffwechselstörungen, die von Diabetes mellitus, Lebererkrankung, Urämie, Nierenerkrankungen oder Entzündung, Heparin, Bakterien und Viren, pathogenbeladenen Blutzellen oder wenigstens Teilen oder Abbauprodukten davon, DNA, Phosphat, Cytokinen, Wachstumsfsktoren, Hormonen, Chemokinen, uramischen Toxinen, Blutgerinnungsproteinen, prokoagulierenden Proteinen, Entzündungs- oder Proentzundungsproteinen, Makrophagenmigration-Hemmfaktor, löslichen oder zelloberflächengebundenen Proteinen, löslichen Adhäsionsmolekülen und Glucose oder Zersetzungsprodukten davon, Pyrogenen, bakteriellen Exotoxinen, Produkten von grampositiven Bakterien, bevorzugt Lipoteichonsäure, insbesondere bakteriellen Pyrogenen, bevorzugt Endotoxinen, insbesondere die Lipid A-Komponente von Lipopolysacchariden (LPS).

8. Polymeraffinitätsmatrix gemäß einem der vorhergehenden Ansprüche, worin die Matrix einen Ausschlusswert von etwa 1 x 10² bis etwa 1 x 10⁶ Dalton hat und hydrophobe und/oder hydrophile Substanzen bindet.

9. Polymeraffinitätsmatrix gemäß einem der vorhergehenden Ansprüche, worin das Fluid, aus welchem die Substanz(en) zu entfernen oder in welchem sie zu verringern ist/sind, eine wässrige oder organische Lösung, ein Körperfluid, bevorzugt Blut, therapeutische Fluide, Fluide für life science-Anivendungen, bevorzugt Pufferlösungen, Infusionsfluide oder Dialysefluide in biologischen, diagnostischen oder biotechnologischen Anwendungen, von gesunden Spendern erhaltene Blutprodukte, wie Plasma, Plättchenkonzentrate, Erythrozytenkonzentrate, die für Transfusionen verwendet werden, Blutersatzprodukte, bevorzugt Sauerstoffträger, modifizierte Hämoglobinlösungen und künstliche Hämoglobinlösungen ist; Fluide für Nahrungszwecke und Fluide für industrielle Verwendung ist.

10. Polymeraffinitätsmatrix gemäß einem der vorhergehenden Ansprüche, worin der feste Träger ein vernetztes Polystyrol ist und die Abstandsgruppe ein Polyethylenglycol ist.

11. Verfahren zum Entfernen einer oder mehrerer Substanzen aus einem Fluid und/oder zum Verringern ihrer Menge oder Konzentration im Hinblick auf das Verhindern, Eliminieren oder Verringern einer unerwünschten Aktivierung von Komponenten oder Vorgängen in dem Fluid, umfassend das Inberührungbringen des Fluids mit der Polymeraffinitätsmatrix, wie in einem der Ansprüche 1 bis 10 definiert, für eine Zeit, die ausreichend ist, um die Menge oder Konzentration der Substanz(en) zu verringern und/oder um sie zu entfernen, bevorzugt bis zu 24 Stunden.

12. Verfahren gemäß Anspruch 11, worin die Zeit 1 Sekunde bis 2 Stunden beträgt.

13. Verfahren gemäß einem der Ansprüche 11 und 12, worin die Substanz ein Endotoxin ist, und das Fluid Blut ist, worin die Menge oder Konzentration von Endotoxin nach dem Entfernen oder Verringern unter der Fähigkeit liegt, Komponenten im Blut zu aktivieren, oder die Aktivierung von Komponenten oder Vorgängen im Blut zu verhindern.

14. Verwendung der Polymeraffinitätsmatrix, wie in einem der Ansprüche 1 bis 10 definiert, zur Entfernung einer oder mehrerer Substanzen, bevorzugt von Endotoxinen, aus einem Fluid, oder zum Erniedrigen ihrer Menge oder Konzentration in dem Fluid, bevorzugt ein Körperfluid oder ein therapeutisches Fluid, am bevorzugtesten Blut oder Serum,

15. Verwendung gemäß Anspruch 14 zur Herstellung von weniger aktiviertem Blut oder zur Verhinderung von unerwünschter Aktivierung von Komponenten oder Vorgängen im Blut.

16. Verwendung gemäß Anspruch 14 als Teil eines extrakorporalen Blutreinigungsverfahrens oder als ein Implantat in dem Körper, um Blut oder irgendein Körperfluid zu kontaktieren, bevorzugt in dem Gefäßsystem, in Blutgefäßen oder in der Bauchfellhöhle.

17. Verwendung gemäß Anspruch 16 zur Herstellung von weniger aktiviertem Blut oder zur Verhinderung von unerwünschter Aktivierung von Komponenten oder Vorgängen im Blut.

18. Kit zum Entfernen einer oder mehrerer Substanzen aus einem Fluid und/oder zum Verringern ihrer Menge oder Konzentration in dem Fluid im Hinblick auf das Verhindern, Eliminieren oder Verringern von unerwünschter Aktivierung von Komponenten oder Vorgängen in dem Fluid, wobei der Kit eine Polymeraffinitätsmatrix, wie in einem der Ansprüche 1 bis 10 definiert, umfasst

19. Kit gemäß Anspruch 18, worin er weiterhin Probeschläuche und eine Vorrichtung für extra- und/oder intrakorporale Behandlung des Fluids, bevorzugt Blut oder Serum, umfasst.

20. Verfahren zum Herstellen einer Polymeraffinitätsmatrix, wie in einem der Ansprüche 1 bis 10 definiert, umfassend
a) Binden der Abstandsgruppe an den festen Träger zum Erhalt eines ersten Komplexes und
b) Binden des wenigstens eine Bindungseinheit mit wenigstens einer funktionellen Gruppe enthaltenden Liganden an den ersten Komplex;
oder
c) Binden der Abstandsgruppe an den wenigstens eine Bindungseinheit mit wenigstens einer funktionellen Gruppe enthaltenden Ligand zum Erhalt eines zweiten Komplexes und
d) Binden des festen Trägers an den zweiten Komplex;
oder
e) Binden der Abstandsgruppe an den festen Träger zum Erhalt eines ersten Komplexes und
f) Festphase-Synthese des Liganden auf der an den festen Träger gebundenen Abstandsgruppe oder
g) Aufbauen oder Synthetisieren der Abstandsgruppe aus Monomeren direkt auf dem festen Träger durch Pfropfen und
h) Binden des wenigstens eine Bindungseinheit mit wenigstens einer funktionellen Gruppe enthaltenden Liganden an den ersten Komplex,
oder
i) Aufbauen oder Synthetisieren der Abstandsgruppe aus Monomeren direkt auf dem festen Träger durch Pfropfen und
k) Festphase-Synthese des Liganden auf der an den festen Träger gebundenen Abstandsgruppe,
worin Information über die dreidimensionale Struktur, die Anwesenheit von Ladungen und von hydrophoben/hydrophilen Bereichen des Bindungsprinzips auf der bzw. den zu bindenden Substanz(en) aus Rönigenstrahl-Kristallografie, Proteinsequenzierung, Proteinmodellierung oder Hydrophobie- und Hydrophilieberechnungen gesammelt wird, und der die Bindungseinheit enthaltende Ligand bezüglich der Ladung und/oder der biydrophme/Hydrophobie zu dem Bindungsprinzip der Substanz(en) komplementär gemacht wird.

21. Verfahren nach Anspruch 20, umfassend die Schritte:
für a) und b), Aktivierung des festen Trägers, Kuppeln des Abstandsgruppemoleküls an den festen Träger, Synthese des die Bindungseinheit enthaltenden Liganden und stellenspezifisches Kuppeln des Liganden an das Abstandsgruppemolekül, oder
für c) und d), Synthese des die Bindungseinheit enthaltenden Liganden, Kuppeln des Abstandsgruppemoleküls an den Ligand, Aktivierung des festen Trägers und stellenspezifisches Kuppeln des Abstandsgruppe-Ligand-Komplexes an den festen Träger, oder
für e) und f), Aktivierung des festen Trägers, Kuppeln des Abstandsgruppemoleküls an den aktivierten festen Träger, und Festphase-Synthese des Liganden auf der an den Träger gebundenen Abstandsgruppe.

22. Verfahren gemäß Anspruch 21, umfassend die Schritte;
für a) und b), Aktivierung der Abstandsgruppe, Kuppeln der aktivierten Abstandsgruppe an den festen Träger und Kuppeln des Liganden an die aktivierte Abstandsgruppe, oder
für c) und d), Synthese des Liganden, Aktivierung der Abstandsgruppe, stellenspezifisches Kuppeln des Liganden an das aktivierte Abstandsgruppemolekül und Kuppeln des Abstandsgruppe-Ligand-Komplexes an den festen Träger oder
für e) und f), Aktivierung der Abstandsgruppe, Kuppeln der aktivierten Abstandsgruppe an den festen Träger und Festphase-Synthese des Liganden auf der an den festen Träger gebundenen Abstandsgruppe.

## Revendications

1. Matrice d'affinité polymère pour lier une ou plusieurs substances dans un fluide pour retirer ladite ou lesdites substances du fluide et/ou diminuer sa quantité ou sa concentration dans ledit fluide dans le but de prévenir, éliminer ou réduire l'activation indésirable de composants ou de processus dans ledit fluide, où ladite matrice comprend
a) un support solide
b) au moins un espaceur lié au support solide, où l'espaceur est choisi dans le groupe consistant en les poly- ou oligo-éthylèneglycols de formule H-(OCH₂CH₂)ₙ-OH, où n représente 2-250, ou les poly(alcools vinyliques), les polyvinylamines, les polyglycidoles, les polyéthylèneimines, les poly(oxydes de propylène) ou leurs dérivés, et, couple à chaque espaceur,
c) ligand ayant une structure tridimensionnelle définie qui est complémentaire en ce qui concerne la charge et/ou l'hydrophobie/hydrophilie de la structure tridimensionnelle d'un motif de liaison de ladite substance ou lesdites substances,
où le ligand est choisi dans le groupe consistant en 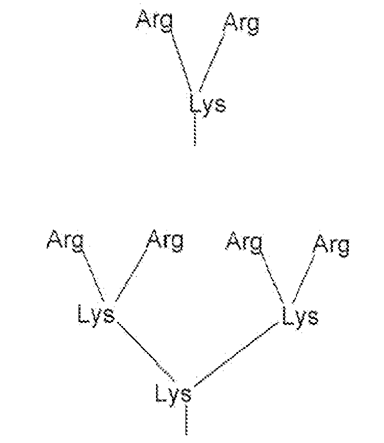 et

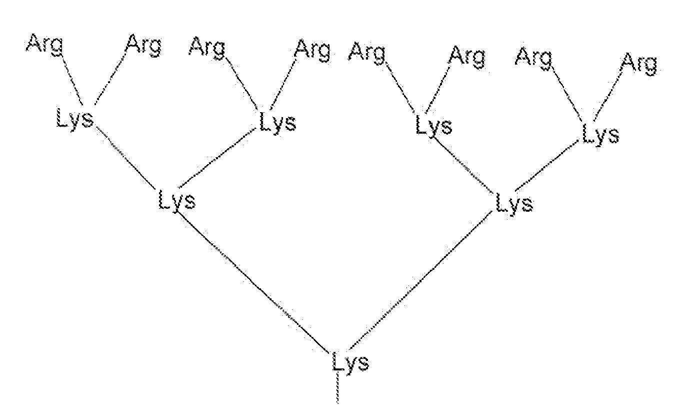

2. Matrice d'affinité polymère selon la revendication 1, où l'espaceur est un polyéthylèneglycol (PEG) dans une configuration linéaire et/ou ramifiée et a une masse moléculaire moyenne de 400-10 000 daltons, ou des dérivés de celui-ci,

3. Matrice d'affinité polymère selon la revendication 1 ou 2, où le support solide est constitué par un matériau choisi dans le groupe consistant en le polystyrène, les poly(alcools vinyliques), les polyhydroxystyrènes, les polymères produits à partir de polystyrènes chlorométhylés ou de polyacrylates, les polyméthacrylates fonctionnalisés avec des groupes hydroxy, les hydroxyalkylpolystyrènes, les hydroxyarylpolystyrènes, les hydroxyalkyl-aryl-polystyrènes, les polystyrènes polyhydroxyalkylés, les polystyrènes polyhydroxyarylés, les isocyanatoalkyl-polystyrènes, les isocyanatoaryl-polystyrèles, les carboxyalkyl-polystyrènes, les carboxyaryl-polystyrènes, les aminoalkyl-polystyrènes, les aminoaryl-polystyrènes, les polyméthacrylates, les polyéthylèneglycols réticulés, la cellulose, la silice, les glucides, un latex, les copolymères de cyclooléfines, le verre ou leurs combinaisons, de préférence un polystyrène réticulé.

4. Matrice d'affinité polymère selon la revendication 3, où le support solide a la forme d'une bille, d'un gel, d'une membrane, d'une particule, d'un filet, d'une étoffe tissée ou non tissée, d'un matelas de fibres, d'un tube, d'un film, d'une feuille ou de combinaisons de ceux-ci ou de réseaux interpénétrants réticulés, de préférence une bille de polystyrène-PEG.

5. Matrice polymère selon l'une quelconque des revendications précédentes, où ladite matrice est biocompatible et a une capacité de gonflement suffisante pour permettre la circulation du sang total.

6. Matrice polymère selon la revendication 5, où la capacité de gonflement est d'environ 1,5-20 fois, de préférence 2-6 fois, d'un état sec à la forme hydratée.

7. Matrice d'affinité polymère selon l'une quelconque des revendications précédentes, où ladite matrice fournit une structure complémentaire tridimensionnelle pour lier au moins une substance choisie dans le groupe consistant en les constituants dérivés de bactéries ou de virus, les endotoxines, les exotoxines, l'ADN bactérien ou des fragments de celui-ci, les oligonucléotides ; les cellules, en particulier les cellules endothéliales, les cellules souches et les tumorales ; les cellules sanguines, en particulier les lymphocytes, les thrombocytes, les granulocytes, les cellules dendritiques et les monocytes; les prions, les parasites, les champignons, les drogues après une surdose, les additifs alimentaires pathogènes, les produits provenant de perturbations métaboliques aiguës ou chroniques résultant du diabète sucré, d'une maladie hépatique, de l'urémie, de maladies rénales ou de l'inflammation, l'héparine, les bactéries et les virus, les cellules sanguines chargées de pathogène, ou au moins des parties ou des produits de dégradation de celles-ci, l'ADN, un phosphate, les cytokines, les facteurs de croissance, les hormones, les chimiokines, les toxines urémiques, les protéines de coagulation du sang, les protéines procoagulantes, les protéines inflammatoires ou pro-inflammatoires, le facteur inhibant la migration des macrophages, les protéines solubles ou liées à la surface cellulaire, les molécules d'adhésion solubles et le glucose ou ses produits de dégradation, les pyrogènes, les exotoxines bactériennes, les produits provenant de bactéries Gram-positives, de préférence l'acide lipotéichonique, en particulier les pyrogènes bactériens, de préférence les endotoxines, en particulier le composant de lipide A des lipopolysaccharides (LPS).

8. Matrice d'affinité polymère selon l'une quelconque des revendications précédentes, où ladite matrice a une valeur de coupure d'environ 1 x 10² à environ 1 x 10⁶ daltons et lie des substances hydrophobes et/ou hydrophiles.

9. Matrice d'affinité polymère selon l'une quelconque des revendications précédentes, où le fluide duquel la ou les substances doivent être retirées ou réduites est une solution aqueuse ou organique, un fluide corporel, de préférence le sang, des fluides thérapeutiques, des fluides pour des applications de science de la vie, de préférence des solutions tampons, des fluides de perfusion ou des fluides de dialyse dans des applications biologiques, diagnostiques ou biotechnologiques, des produits sanguins obtenus à partir de donneurs sains, comme le plasma, des concentrés de plaquettes, des concentrés d'érythrocytes qui sont utilisés pour les transfusions, des produits de remplacement du sang, de préférence des vecteurs d'oxygène, des solutions d'hémoglobine modifiée et des solutions d'hémoglobine artificielle ; des fluides pour la nutrition et des fluides pour l'utilisation industrielle.

10. Matrice d'affinité polymère selon l'une quelconque des revendications précédentes, où le support solide est un polystyrène réticulé, et l'espaceur est un polyéthylèneglycol.

11. Procédé pour retirer une ou plusieurs substances d'un fluide et/ou réduire sa quantité ou sa concentration dans le but de prévenir, éliminer ou réduire l'activation indésirable de composants ou de processus dans ledit fluide, comprenant la mise en contact du fluide avec la matrice d'affinité polymère selon l'une quelconque des revendications 1-10 pendant une durée suffisante pour réduire la quantité ou la concentration et/ou retirer ladite ou lesdites substances, de préférence de jusqu'à 24 h.

12. Procédé selon la revendication 11, où la durée est de 1 s à 2 h.

13. Procédé selon l'une quelconque des revendications 11 et 12, où la substance est une endotoxine et le fluide est le sang, où la quantité ou la concentration d'endotoxine après avoir été retirée ou réduite est inférieure à la capacité d'activation de composants dans le sang ou prévient l'activation de composants ou de processus dans le sang.

14. Utilisation de la matrice d'affinité polymère selon l'une quelconque des revendications 1-10 pour retirer une ou plusieurs substances, de préférence des endotoxines, d'un fluide, ou pour diminuer sa quantité ou sa concentration dans ledit fluide, de préférence un fluide corporel ou un fluide thérapeutique, de manière particulièrement préférable le sang ou le sérum.

15. Utilisation selon la revendication 14 pour la production de sang moins activé ou la prévention de l'activation indésirable de composants ou de processus dans le sang.

16. Utilisation selon la revendication 14 dans le cadre d'un procédé de purification du sang extracorporel ou sous forme d'un implant dans le corps destiné à entrer en contact avec le sang ou tout fluide corporel, de préférence dans le système vasculaire, des vaisseaux sanguins ou la cavité péritonéale.

17. Utilisation selon la revendication 16, pour la production de sang moins activé ou la prévention d'une activation indésirable de composants ou de processus dans le sang.

18. Kit pour retirer une ou plusieurs substances d'un fluide et/ou diminuer sa quantité ou sa concentration dans ledit fluide dans le but de prévenir, éliminer ou réduire l'activation indésirable de composants ou de processus dans ledit fluide, ledit kit comprenant une matrice d'affinité polymère selon l'une quelconque des revendications 1-10.

19. Kit selon la revendication 18, où il comprend en outre des tubes à échantillon, et un dispositif pour le tracement extra- et/ou intracorporel dudit fluide, de préférence le sang ou le sérum.

20. Procédé pour produire une matrice d'affinité polymère selon l'une quelconque des revendications 1-10, comprenant
a) la fixation de l'espaceur au support solide pour obtenir un premier complexe, et
b) la fixation audit premier complexe du ligand contenant ladite au moins une unité de liaison avec au moins un groupe fonctionnel ;
ou
c) la fixation de l'espaceur au ligand contenant ladite au moins une unité de liaison avec au moins un groupe fonctionnel pour obtenir un second complexe, et
d) la fixation du support solide audit second complexe
ou
e) la fixation de l'espaceur au support solide pour obtenir un premier complexe, et
f) la synthèse en phase solide du ligand sur l'espaceur lié au support solide, ou
g) la construction ou la synthèse de l'espaceur à partir de monomères directement sur le support solide par greffage, et
h) la fixation audit premier complexe du ligand contenant ladite au moins une unité de raison avec au moins un groupe fonctionnel,
ou
i) la construction ou la synthèse de l'espaceur à partir de monomères directement sur le support solide par greffage, et
k) la synthèse en phase solide du ligand sur l'espaceur lié au support solide,
où les informations concernant la structure tridimensionnelle, la présence de charges et de régions hydrophobes/hydrophiles du motif de liaison sur la ou les substances destinées à être liées sont recueillies d'après la cristallographie des rayons X, le séquençage des protéines, la modélisation des protéines ou les calculs d'hydrophobie et d'hydrophilie et le ligand contenant l'unité de liaison est rendu complémentaire en ce qui concerne la charge et/ou l'hydrophilie/hydrophobie du motif de liaison de ladite substance ou desdites substances.

21. Procédé selon la revendication 20 comprenant les étapes de
pour a) et b), activation du support solide, couplage de la molécule d'espaceur sur le support solide, synthèse du ligand contenant l'unité de liaison, et couplage spécifique de site du ligand à la molécule d'espaceur, ou
pour c) et d), synthèse du ligand contenant l'unité de liaison, couplage de la molécule d'espaceur au ligand, activation du support solide, et couplage spécifique de site du complexe espaceur-ligand au support solide, ou
pour e) et f), activation du support solide, couplage de la molécule d'espaceur au support solide activé, et synthèse en phase solide du ligand sur l'espaceur lié au support.

22. Procédé selon la revendication 21, comprenant les étapes de
pour a) et b), activation de l'espaceur, couplage de l'espaceur activé au support solide, et couplage du ligand audit espaceur activé, ou
pour c) et d), synthèse du ligand, activation de l'espaceur, couplage spécifique de site du ligand à la molécule d'espaceur activé et couplage du complexe espaceur-ligand au support solide, ou
pour e) et f), activation de l'espaceur, couplage de l'espaceur activé au support solide et synthèse en phase solide du ligand sur l'espaceur lié au support solide.
